(19) Europäisches Patentamt
European Patent Office
Office européen des brevets

(11) **EP 2 635 261 B1**

(12) **EUROPEAN PATENT SPECIFICATION**

(45) Date of publication and mention
of the grant of the patent:
**23.10.2019 Bulletin 2019/43**

(21) Application number: **11838933.7**

(22) Date of filing: **07.11.2011**

(51) Int Cl.:
*A61K 9/51* *(2006.01)*          *A61K 48/00* *(2006.01)*
*C12N 15/88* *(2006.01)*

(86) International application number:
**PCT/US2011/059558**

(87) International publication number:
**WO 2012/061803 (10.05.2012 Gazette 2012/19)**

(54) **COMPOSITIONS AND METHODS FOR NANOPOLYMER-BASED NUCLEIC ACID DELIVERY**

ZUSAMMENSETZUNGEN UND VERFAHREN FÜR NANOPOLYMERBASIERTE FREISETZUNG VON NUKLEINSÄUREN

COMPOSITIONS ET MÉTHODES UTILISABLES EN VUE DE L'ADMINISTRATION D'ACIDES NUCLÉIQUES, FAISANT APPEL À DES NANOPOLYMÈRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.11.2010 US 411358 P**
**06.11.2010 US 410863 P**

(43) Date of publication of application:
**11.09.2013 Bulletin 2013/37**

(73) Proprietor: **MARINE POLYMER TECHNOLOGIES, INC.**
**Burlington, MA 01803 (US)**

(72) Inventors:
• **VOURNAKIS, John, N.**
**deceased (US)**

• **DEMCHEVA, Marina, V.**
**Tewksbury**
**MA 01876 (US)**

(74) Representative: **Jones Day**
**Rechtsanwälte, Attorneys-at-Law, Patentanwälte**
**Prinzregentenstrasse 11**
**80538 München (DE)**

(56) References cited:
**US-A1- 2003 104 020     US-A1- 2006 105 049**
**US-A1- 2007 085 059     US-A1- 2008 207 561**
**US-A1- 2010 040 694     US-A1- 2010 086 613**
**US-A1- 2010 150 960**

**Description**

**1. INTRODUCTION**

Provided herein is a poly-N-acetylglucosamine nanoparticle/nucleic acid composition for use in a method of treating or preventing cancer, an infectious disease, or a genetic deficiency of a necessary protein, wherein the poly-N-acetylglucosamine nanoparticle/nucleic acid composition comprises poly-N-acetylglucosamine and the nucleic acid, wherein the nanoparticles are between about 5 nm and 500 nm in size, and wherein 40% to 80% of the poly-N-acetylglucosamine is deacetylated; and wherein the method comprises administering the poly-N-acetylglucosamine nanoparticle/nucleic acid composition subcutaneously to the subject.

Further disclosed are p-GlcNAc nanoparticle/nucleic acid compositions comprising deacetylated poly-N-acetylglucosamine lactate derivative nanoparticles less than 500 nm and a nucleic acid. Also, disclosed herein are methods for administering a nucleic acid to a subject, the method comprising administering to the subject a p-GlcNAc nanoparticle/nucleic acid composition.

**2. BACKGROUND**

DNA vaccines represent a flexible strategy that precisely and effectively presents antigens to the immune system. However, despite all the theoretical advantages of DNA vaccines, the clinical experience with DNA vaccines has been rather disappointing. It is becoming increasingly evident that one of the central problems in clinical translation of DNA vaccines is suboptimal platforms for plasmid DNA delivery. Further, improved platforms of nucleic acid delivery are required for a wide variety of *in vivo* and *ex vivo* gene therapy applications.

Viral platforms for DNA delivery, such that based on retroviruses and adenoviruses, have been developed. However, viral vectors have significant disadvantages. Administration of recombinant viruses induces an immune response to viral proteins, a response that may be about 20-fold higher than that induced by the transgene (see Harrington et al., 2002, Hum. Gene Ther. 13(11):1263-1280; and Harrington et al., 2002, J. Virol. 76(7):3329-3337). Such immune response limits the immune response to the transgene itself. The pre-existence of T-cell and antibody-mediated immunity to viral particles also limits the ability of subsequent administration of recombinant viruses (see Barouch et al., 2003, J. Virol. 77(13):7367-7375; Premenko-Lanier et al., 2003, Virology 307(1):67-75; Ramirez et al., 2000, J. Virol. 74(16):7651-7655; and Ramirez et al., 2000, J. Virol. 74(2):923-933), and thus does not allow for repeated treatment regimens. The repeated administration of such vector leads to generation of neutralizing antibodies against them (see Tewary et al., 2005, J. Infect. Dis. 191(12):2130-2137). Although the repeated delivery may be accomplished by use of different viral vectors, such approach is laborious and requires preparation of large amounts of different viral vectors raising biosafety concerns. In addition, viral delivery platforms create a risk of interaction of viral genetic sequences with those of a host genome. It is also known that most of the viral vectors are degraded by serum nucleases such that almost 90% of injected viral vectors are degraded within 24 hours (see Muzyczka, 1992, Curr. Top. Microbiol. Immunol. 158:97-129; and Varmus, 1988, Science 240(4858):1427-1435). The fast degradation of the viral vectors may result in their failure to reach the target cells. Taken together, viral platforms for nucleic acid delivery have significant limitations.

Non-viral platforms for DNA delivery, including liposomes (lipoplex), synthetic polymers (polyplex) (see Wasungu et al., 2006, J. Control. Release 116(2):255-264; and Wasungu, 2006, Biochim. Biophys. Acta 1758(10):1677-1684), and chitosan (see Mansouri et al. 2004, Eur. J. Pharm. Biopharm. 57(1):1-8), also have proven to be suboptimal. The preparation of lipoplexes is very demanding and requires formulation of DNA into the vehicle (see Wasungu et al., 2006, J. Control. Release 116(2):255-264; and Wasungu, 2006, Biochim. Biophys. Acta 1758(10):1677-1684). In addition, publications by Wasungu et al. show that several physical factors such as pH and charge and the structural characteristics of liposomes affect interactions of liposomes with DNA, and that lipoplexes achieve low transduction efficiency due to their rapid clearance from the circulation. The process of lipoplex and polyplex assembly could compromise the structural integrity of the plasmid DNA, such that the resulting inefficient wrapping of plasmid into the lipoplex shell can affect interaction of lypoplexes with cell surfaces. This can result in a very poor transcription of lipoplex- or polyplex- delivered genes (see Hama, 2006, Mol. Ther. 13(4):786-794). Further, most of the polyplexes require co-transfection with endosome-lytic agents because of their inability to release intracellular DNA into the cytoplasm (see Forrest and Pack, 2002, Mol. Ther.. 6(1):57-66).

Use of chitin and chitosan-based products for DNA delivery applications has been hampered by the chemical and physical heterogeneity of the polymer products and contamination of chitin and chitosan preparations by proteins and other components (see Vournakis et al., 2004, J. Trauma 57(1 Suppl.):S2-6).

US2010/0086613 discloses chitosan-DNA nanoparticles, wherein at least 81 % of the poly-N-acetylglucosamine is deacetylated.

Accordingly, there is a need for a non-viral platform for nucleic acid delivery that can induce high transfection efficiency but without inducing toxicity. There is also a need for a delivery vehicle that would allow for sustained release of nucleic acids, allowing for repeated administration but reducing its frequency.

## 3. SUMMARY

Provided herein are poly-N-acetylglucosamine ("p-GlcNAc") nanoparticle/nucleic acid compositions. In one aspect, provided herein is a poly-N-acetylglucosamine nanoparticle/nucleic acid composition for use in a method of treating or preventing cancer, an infectious disease, or a genetic deficiency of a necessary protein, wherein the poly-N-acetylglucosamine nanoparticle/nucleic acid composition comprises poly-N-acetylglucosamine and the nucleic acid, wherein the nanoparticles are between about 5 nm and 500 nm in size, and wherein 40% to 80% of the poly-N-acetylglucosamine is deacetylated; and wherein the method comprises administering the poly-N-acetylglucosamine nanoparticle/nucleic acid composition subcutaneously to the subject. In some embodiments, the subject is human. In other embodiments, the subject is a non-human animal. In some embodiments the administering of the poly-N-acetylglucosamine nanoparticle/nucleic acid composition results in a sustained expression of the nucleic acid for at least 1 week, 2 weeks, or 4 weeks at the site of administration to the subject. In some embodiments, the method comprises repeated administration. In some embodiments, the poly-N-acetylglucosamine nanoparticle/nucleic acid composition further comprises an adjuvant, preferably the adjuvant is a cytokine or a polyinosinic:polycytidylic acid ("poly I:C"). In a further embodiment, the poly-N-acetylglucosamine nanoparticle/nucleic acid composition provides sustained concurrent release of both the nucleic acid and the adjuvant. In some embodiments, the deacetylated poly-N-acetylglucosamine comprises a deacetylated poly-N-acetylglucosamine ammonium salt derivative, preferably a deacetylated poly-N-acetylglucosamine lactate derivative; or the deacetylated poly-N-acetylglucosamine has been solubilized with an organic or mineral acid, preferably with a lactic acid. In some embodiments, at least 50% of the nanoparticles are between 20 nm and 200 nm, or between 25 nm and 150 nm in size. In some embodiments, the size is determined by transmission electron microscopy or scanning electron microscopy. In some embodiments, the method is part of a gene therapy protocol or vaccination protocol. In some embodiments, the poly-N-acetylglucosamine is 60% to 80% deacetylated. In a specific embodiment, the poly-N-acetylglucosamine is 65% to 75% deacetylated. In some embodiments, the nucleic acid is DNA. In a further aspect, provided herein is a method of making a poly-N-acetylglucosamine nanoparticle/nucleic acid composition comprising:

(a) adding a base to poly-N-acetylglucosamine to deacetylate at least 40% to 80% of the poly-N-acetylglucosamine;
(b) adding a lactic acid to a form a deacetylated poly-N-acetylglucosamine lactate derivative;
(c) adding a buffer to facilitate dilution; and
(d) adding a nucleic acid, thereby making a poly-N-acetylglucosamine nanoparticle/nucleic acid composition.

In some embodiments, the buffer in step (c) is sodium acetate-acetic buffer.
In some embodiments, the nucleic acid has been combined with a salt, preferably sodium sulfate.
In some embodiments, the method further comprises:

(i) adding an adjuvant in step (d); or
(ii) combining the poly-N-acetylglucosamine nanoparticle/nucleic acid composition with an adjuvant,

wherein the adjuvant is preferably poly I:C.
In some embodiments, the poly-N-acetylglucosamine is 60% to 80% deacetylated. In a specific embodiment, the poly-N-acetylglucosamine is 65% to 75% deacetylated. In some embodiments, the nucleic acid is DNA.

It is also disclosed herein that p-GlcNAc nanoparticle/nucleic acid compositions comprise poly-N-acetylglucosamine and a nucleic acid, wherein at least 40% of the poly-N-acetylglucosamine is deacetylated. In some embodiments, the nucleic acid is DNA. In certain embodiments, the nanoparticles in the p-GlcNAc nanoparticle/nucleic acid compositions are between about 5 nm and 500 nm in size. In some embodiments, at least 50% of the nanoparticles in the p-GlcNAc nanoparticle/nucleic acid compositions are between about 5 nm and 500 nm in size. In certain embodiments, the nanoparticles in the p-GlcNAc nanoparticle/nucleic acid compositions are between about 10 nm and 500 nm, 20 nm and 200 nm, 20 nm and 150 nm, 20 nm and 100 nm, 25 nm to 250 nm, 25 nm and 150 nm, 25 nm and 100 nm, 50 nm and 200 nm, or 50 nm and 150 nm in size. In specific embodiments, at least 50% of the nanoparticles in the p-GlcNAc nanoparticle/nucleic acid compositions are between about 10 nm and 500 nm, 20 nm and 200 nm, 20 nm and 150 nm, 20 nm and 100 nm, 25 nm to 250 nm, 25 nm and 150 nm, 25 nm and 100 nm, 50 nm and 200 nm, or 50 nm and 150 nm in size. In particular embodiments, at least 60%, 70%, 80%, 90%, 95%, 98%, or 99% of the nanoparticles are between 5 nm and 500 nm, 10 nm and 500 nm, 20 nm and 200 nm, 20 nm and 150 nm, 20 nm and 100 nm, 25 nm to 250 nm, 25 nm and 150 nm, 25 nm and 100 nm, 50 nm and 200 nm, or 50 nm and 150 nm in size. In certain embodiments, the size of the nanoparticles is determined by transmission electron microscopy or scanning electron microscopy. In some embodiments, the composition further comprises an adjuvant. In a specific embodiment, the adjuvant is PolyI:C. In another embodiment, the adjuvant is a cytokine.

In some embodiments, the deacetylated poly-N-acetylglucosamine in the p-GlcNAc nanoparticle/nucleic acid composition comprises a deacetylated poly-N-acetylglucosamine ammonium salt derivative. In a specific embodiment, the deacetylated poly-N-acetylglucosamine in the composition comprises a deacetylated poly-N-acetylglucosamine lactate derivative. In some embodiments, the deacetylated poly-N-acetylglucosamine in the composition has been solubilized with an organic or mineral acid. In a specific embodiment, the deacetylated poly-N-acetylglucosamine in the composition has been solubilized with a lactic acid.

In certain examples, described herein are p-GlcNAc nanoparticle/nucleic acid compositions wherein at least 65% of the poly-N-acetylglucosamine is deacetylated. In some examples, at least 70% of the poly-N-acetylglucosamine in the p-GlcNAc nanoparticle/nucleic acid composition is deacetylated. In one embodiment, about 60% to about 80% (e.g., 60% to 80%) of the poly-N-acetylglucosamine in the composition is deacetylated. In other embodiments, about 40% to about 80%, about 50% to about 80%, about 55% to about 80%, about 65% to about 80%, or about 65% to about 75% of the poly-N-acetylglucosamine in the composition is deacetylated. In other examples, about 40% to about 90% (e.g., 40% to 90%) of the poly-N-acetylglucosamine in the composition is deacetylated. In other examples, about 40% to about 95%, about 40% to about 85%, about 50% to about 95%, about 50% to about 90%, about 50% to about 85%, about 55% to about 95%, about 55% to about 90%, about 55% to about 85%, about 60% to about 95%, about 60% to about 90%, about 60% to about 85%, about 65% to about 95%, about 65% to about 90%, or about 65% to about 85% of the poly-N-acetylglucosamine in the composition is deacetylated.

In some embodiments, the poly-N-acetylglucosamine in the p-GlcNAc nanoparticle/nucleic acid composition is a fiber of about 50 to about 200 $\mu$m in length. In a specific embodiment, the poly-N-acetylglucosamine in the p-GlcNAc nanoparticle/nucleic acid composition is a fiber of 50 to 100 $\mu$m in length. In some embodiments, the poly-N-acetylglucosamine in the p-GlcNAc nanoparticle/nucleic acid composition has a molecular weight of at least $2 \times 10^6$ Da or at least $2.5 \times 10^6$ Da, or molecular weight between about $2 \times 10^6$ Da and about $3.5 \times 10^6$ Da, or between about $2.5 \times 10^6$ Da and about $3 \times 10^6$ Da.

It is also disclosed herein that the p-GlcNAc nanoparticle/nucleic acid compositions comprise deacetylated poly-N-acetylglucosamine lactate derivative nanoparticles less than 500 nm and a nucleic acid. In certain embodiments, at least 50%, 60%, 65%, 70%, 75%, 80%, 85% or 90% of the nanoparticles are 100 to 200 nm in size as determined by, *e.g.,* transmission electron microscopy or scanning electron microscopy. In some embodiments, the composition further comprises an adjuvant. In a specific embodiment, the adjuvant is PolyI:C. In another embodiment, the adjuvant is a cytokine.

Disclosed herein are methods for administering a nucleic acid to a subject, the method comprising administering to the subject a p-GlcNAc nanoparticle/nucleic acid composition. In some embodiments, the subject is a human. In other embodiments, the subject is a non-human animal. In certain embodiments, the p-GlcNAc nanoparticle/nucleic acid composition is administered subcutaneously to the subject. In specific embodiments, the p-GlcNAc nanoparticle/nucleic acid composition is administered subcutaneously to epithelial cells of a subject. In other embodiments, the p-GlcNAc nanoparticle/nucleic acid composition is administered intramuscularly or intravenously to a subject. The methods described herein are based, at least in part, on the surprising discovery that the administration of p-GlcNAc nanoparticle/nucleic acid compositions to a subject result in sustained expression of nucleic acid at the site of administration. In addition, the expressed nucleic acid can be effectively taken up by professional antigen-presenting cells and transported to the draining lymph nodes which results in specific CD8+ T cell activity. In some embodiments, the administration of the composition results in a sustained expression of the nucleic acid in the composition for at least 1 week, at least 2 weeks, at least 4 weeks, at least 6 weeks, or at least 2 months. In certain embodiments, a p-GlcNAc nanoparticle/nucleic acid composition is repeatedly administered to a subject (e.g., twice, three times, four times, or more than three or four times; or once a week, once in 2 weeks, once in 3 weeks, once in 4 weeks, once in 6 weeks, once in 8 weeks). In some embodiments, the p-GlcNAc nanoparticle/nucleic acid composition is repeatedly administered over a period of 1 month, 2 months, 3 months, 4 months, 5 months, 6 months, 7 months, 8 months, 9 months, 10 months, 11 months, 12 months, 2 years, 3 years, 4 years or 5 years (or more than 1 year or 5 years).

Disclosed herein are methods for administering a nucleic acid and an adjuvant to a subject, the method comprising administering to the subject a p-GlcNAc nanoparticle/nucleic acid composition and an adjuvant. The adjuvant can be administered in the p-GlcNAc nanoparticle/nucleic acid composition, or administered concomitantly with the p-GlcNAc nanoparticle/nucleic acid composition (e.g., in a separate composition comprising p-GlcNAc and an adjuvant). In some embodiments, administering of the p-GlcNAc nanoparticle/nucleic acid composition comprising a nucleic acid and an adjuvant results in a sustained concurrent release of both the nucleic acid and the adjuvant.

Disclosed herein are methods of making a poly-N-acetylglucosamine nanoparticle/nucleic acid composition. In particular, described herein are methods of making the composition comprising: (a) adding a base to poly-N-acetylglucosamine to deacetylate at least 40% of the poly-N-acetylglucosamine; (b) adding a mineral acid or organic acid to form a deacetylated poly-N-acetylglucosamine ammonium salt derivative; (c) adding a buffer to facilitate dilution; and (d) adding a nucleic acid; thereby making a poly-N-acetylglucosamine nanoparticle/nucleic acid composition. In one embodiment, the mineral acid or organic acid is lactic acid. In some examples, the mineral acid or organic acid is glycolic, succinic, citric, gluconic, glucoronic, malic, pyruvic, tartaric, tartronic or fumaric acid. In specific embodiments, the buffer in step (c) is sodium acetate-acetic buffer or ammonium acetate-acetic buffer. In some embodiments, the nucleic acid has been combined with a salt (e.g., sodium sulfate, potassium sulfate, calcium sulfate or magnesium sulfate) prior to step (d) (in which nucleic acid is added to the deacetylated poly-N-acetylglucosamine ammonium salt derivative diluted in a buffer). In certain embodiments, the poly-N-acetylglucosamine used in making the poly-N-acetylglucosamine nanoparticle/nucleic acid compositions is 60% to 80% deacetylated. In some specific embodiments, the poly-N-acetylglucosamine used in making the described compositions is about 40% to about 80%, about 50% to about 80%, about 55% to about 80%, about 65% to about 80%, or about 65% to about 75% deacetylated.

In certain examples, the poly-N-acetylglucosamine used in making the poly-N-acetylglucosamine nanoparticle/nucleic acid compositions is 40% to 90% deacetylated or more than 65% deacetylated. In some specific examples, the poly-N-acetylglucosamine used in making the described compositions is about 40% to about 95%, about 40% to about 85%, about 50% to about 95%, about 50% to about 90%, about 50% to about 85%, about 55% to about 95%, about 55% to about 90%, about 55% to about 85%, about 60% to about 95%, about 60% to about 90%, about 60% to about 85%, about 65% to about 95%, about 65% to about 90%, or about 65% to about 85% deacetylated.

In certain embodiments, described herein are methods of making the poly-N-acetylglucosamine nanoparticle/nucleic acid composition, which further comprises adding an adjuvant in step (d) described above. Yet in other embodiments, described herein are methods of making the poly-N-acetylglucosamine nanoparticle/nucleic acid composition, which further comprises combining the poly-N-acetylglucosamine nanoparticle/nucleic acid composition with an adjuvant. The adjuvant can be any adjuvant described herein (e.g., poly I:C or a cytokine).

### 3.1 Terminology

The term "alkyl" refers to a linear or branched saturated monovalent hydrocarbon radical, wherein the alkylene may optionally be substituted as described herein. The term "alkyl" also encompasses both linear and branched alkyl, unless otherwise specified. In certain embodiments, the alkyl is a linear saturated monovalent hydrocarbon radical that has 1 to 20 ($C_{1-20}$), 1 to 15 ($C_{1-15}$), 1 to 10 ($C_{1-10}$), or 1 to 6 ($C_{1-6}$) carbon atoms, or branched saturated monovalent hydrocarbon radical of 3 to 20 ($C_{3-20}$), 3 to 15 ($C_{3-15}$), 3 to 10 ($C_{3-10}$), or 3 to 6 ($C_{3-6}$) carbon atoms. As used herein, linear $C_{1-6}$ and branched $C_{3-6}$ alkyl groups are also referred as "lower alkyl." Examples of alkyl groups include, but are not limited to, methyl, ethyl, propyl (including all isomeric forms), n-propyl, isopropyl, butyl (including all isomeric forms), n-butyl, isobutyl, *sec*-butyl, t-butyl, pentyl (including all isomeric forms), and hexyl (including all isomeric forms). For example, $C_{1-6}$ alkyl refers to a linear saturated monovalent hydrocarbon radical of 1 to 6 carbon atoms or a branched saturated monovalent hydrocarbon radical of 3 to 6 carbon atoms.

The term "alkenyl" refers to a linear or branched monovalent hydrocarbon radical, which contains one or more, in one embodiment, one to five, carbon-carbon double bonds. The alkenyl may be optionally substituted as described herein. The term "alkenyl" also embraces radicals having "*cis*" and "*trans*" configurations, or alternatively, "Z" and "E" configurations, as appreciated by those of ordinary skill in the art. As used herein, the term "alkenyl" encompasses both linear and branched alkenyl, unless otherwise specified. For example, $C_{2-6}$ alkenyl refers to a linear unsaturated monovalent hydrocarbon radical of 2 to 6 carbon atoms or a branched unsaturated monovalent hydrocarbon radical of 3 to 6 carbon atoms. In certain embodiments, the alkenyl is a linear monovalent hydrocarbon radical of 2 to 20 ($C_{2-20}$), 2 to 15 ($C_{2-15}$), 2 to 10 ($C_{2-10}$), or 2 to 6 ($C_{2-6}$) carbon atoms, or a branched monovalent hydrocarbon radical of 3 to 20 ($C_{3-20}$), 3 to 15 ($C_{3-15}$), 3 to 10 ($C_{3-10}$), or 3 to 6 ($C_{3-6}$) carbon atoms. Examples of alkenyl groups include, but are not limited to, ethenyl, propen-1-yl, propen-2-yl, allyl, butenyl, and 4-methylbutenyl.

The term "alkynyl" refers to a linear or branched monovalent hydrocarbon radical, which contains one or more, in one embodiment, one to five, carbon-carbon triple bonds. The alkynyl may be optionally substituted as described herein. The term "alkynyl" also encompasses both linear and branched alkynyl, unless otherwise specified. In certain embodiments, the alkynyl is a linear monovalent hydrocarbon radical of 2 to 20 ($C_{2-20}$), 2 to 15 ($C_{2-15}$), 2 to 10 ($C_{2-10}$), or 2 to 6 ($C_{2-6}$) carbon atoms, or a branched monovalent hydrocarbon radical of 3 to 20 ($C_{3-20}$), 3 to 15 ($C_{3-15}$), 3 to 10 ($C_{3-10}$), or 3 to 6 ($C_{3-6}$) carbon atoms. Examples of alkynyl groups include, but are not limited to, ethynyl (-C≡CH) and propargyl (-CH$_2$C≡CH). For example, $C_{2-6}$ alkynyl refers to a linear unsaturated monovalent hydrocarbon radical of 2 to 6 carbon atoms or a branched unsaturated monovalent hydrocarbon radical of 3 to 6 carbon atoms.

The term "halogen", "halide" or "halo" refers to fluorine, chlorine, bromine, and/or iodine.

The term "optionally substituted" is intended to mean that a group, such as an alkyl, alkenyl, alkynyl, cycloalkyl, aryl, heteroaryl, heterocyclyl, or alkoxy group, may be substituted with one or more substituents independently selected from, e.g., (a) alkyl, alkenyl, alkynyl, cycloalkyl, aryl, heteroaryl, and heterocyclyl, each optionally substituted with one or more, in one embodiment, one, two, three, or four, substituents Q; and (b) halo, cyano (-CN), nitro (-NO$_2$), -C(O)R$^a$, -C(O)OR$^a$,

-C(O)NR$^b$R$^c$, -C(NR$^a$)NR$^b$R$^c$, -OR$^a$, -OC(O)R$^a$, -OC(O)OR$^a$, -OC(O)NR$^b$R$^c$, -OC(=NR$^a$)NR$^b$R$^c$, -OS(O)R$^a$, -OS(O)$_2$R$^a$, -OS(O)NR$^b$R$^c$, -OS(O)$_2$NR$^b$R$^c$, -NR$^b$R$^c$, -NR$^a$C(O)R$^d$, -NR$^a$C(O)OR$^d$, -NR$^a$C(O)NR$^b$R$^c$, -NR$^a$C(=NR$^d$)NR$^b$R$^c$, -NR$^a$S(O)R$^d$, -NR$^a$S(O)$_2$R$^d$, -NR$^a$S(O)NR$^b$R$^c$, -NR$^a$S(O)$_2$NR$^b$R$^c$, -SR$^a$, -S(O)R$^a$, -S(O)$_2$R$^a$, -S(O)NR$^b$R$^c$, and -S(O)$_2$NR$^b$R$^c$, wherein each R$^a$, R$^b$, R$^c$, and R$^d$ is independently (i) hydrogen; (ii) C$_{1-6}$ alkyl, C$_{2-6}$ alkenyl, C$_{2-6}$ alkynyl, C$_{3-7}$ cycloalkyl, C$_{6-14}$ aryl, heteroaryl, or heterocyclyl, each optionally substituted with one or more, in one embodiment, one, two, three, or four, substituents Q; or (iii) R$^b$ and R$^c$ together with the N atom to which they are attached form heterocyclyl, optionally substituted with one or more, in one embodiment, one, two, three, or four, substituents Q. As used herein, all groups that can be substituted are "optionally substituted," unless otherwise specified.

In one embodiment, each Q is independently selected from the group consisting of (a) cyano, halo, and nitro; and (b) C$_{1-6}$ alkyl, C$_{2-6}$ alkenyl, C$_{2-6}$ alkynyl, C$_{3-7}$ cycloalkyl, C$_{6-14}$ aryl, heteroaryl, and heterocyclyl; and -C(O)R$^e$, -C(O)OR$^e$, -C(O)NR$^f$R$^g$, -C(NR$^e$)NR$^f$R$^g$, -OR$^e$, -OC(O)R$^e$, -OC(O)OR$^e$, -OC(O)NR$^f$R$^g$, -OC(=NR$^e$)NR$^f$R$^g$, -OS(O)R$^e$, -OS(O)$_2$R$^e$, -OS(O)NR$^f$R$^g$, -OS(O)$_2$NR$^f$R$^g$, -NR$^f$R$^g$, -NR$^e$C(O)R$^h$, -NR$^e$C(O)OR$^h$, -NR$^e$C(O)NR$^f$R$^g$, -NR$^e$C(=NR$^h$)NR$^f$R$^g$, -NR$^e$S(O)R$^h$, -NR$^e$S(O)$_2$R$^h$, -NR$^e$S(O)NR$^f$R$^g$, -NR$^e$S(O)$_2$NR$^f$R$^g$, -SR$^e$, -S(O)R$^e$, -S(O)$_2$R$^e$, -S(O)NR$^f$R$^g$, and -S(O)$_2$NR$^f$R$^g$; wherein each R$^e$, R$^f$, R$^g$, and R$^h$ is independently (i) hydrogen; (ii) C$_{1-6}$ alkyl, C$_{2-6}$ alkenyl, C$_{2-6}$ alkynyl, C$_{3-7}$ cycloalkyl, C$_{6-14}$ aryl, heteroaryl, or heterocyclyl; or (iii) R$^f$ and R$^g$ together with the N atom to which they are attached form heterocyclyl.

## 4. BRIEF DESCRIPTION OF FIGURES

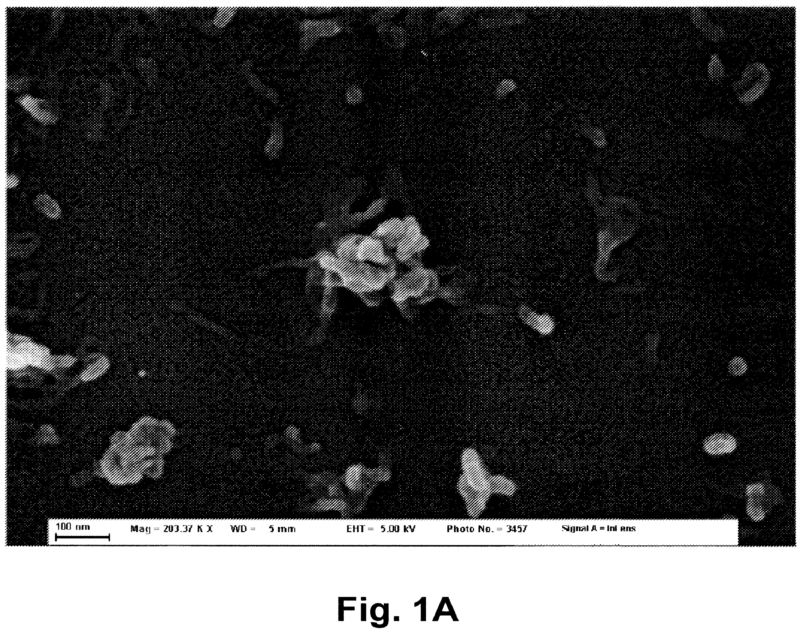

Figure 1 (A)-(F). Scanning electron micrographs of p-GlcNAc nanoparticles.

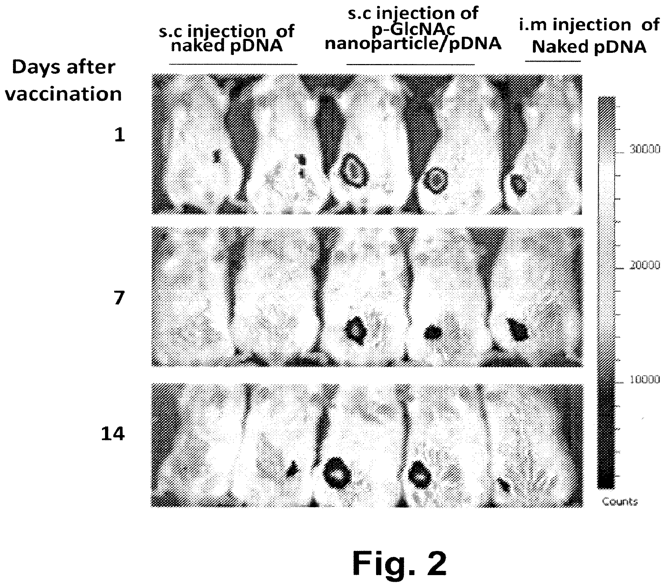

Figure 2. Bioluminescent detection of luciferase activity after vaccination with plasmid DNA encoding luciferase with or without p-GlcNAc nanoparticles. Plasmid DNA encoding the gene for luciferase was delivered as indicated (i.e., subcutaneous injection of naked pDNA; subcutaneous injection of p-GlcNAc nanoparticle/pDNA, or intramuscular injection of naked DNA). Luciferase activity was detected after intraperitoneal (i.p) injection of luciferin at the indicated time interval. Bioluminescence was imaged using the IVIS system. p-GlcNAc nanoparticles effectively release DNA 1, 7 and 14 days after subcutaneous injection.

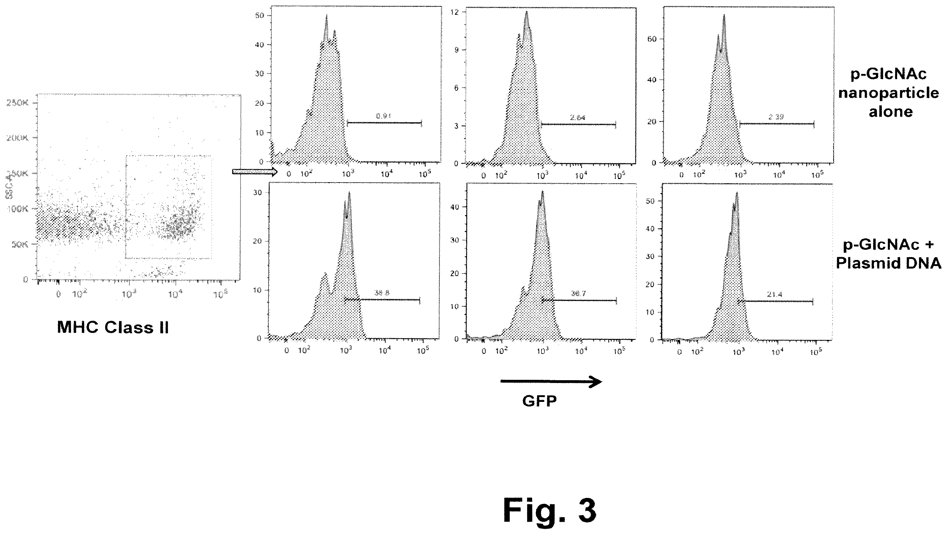

Figure 3. Delivery of DNA using p-GlcNAc nanoparticle/DNA composition results in uptake and transport of encoded antigen to draining lymph node by professional antigen presenting cells. Six mice were injected in the footpad with p-GlcNAc nanoparticles alone (n=3) or with p-GlcNAc nanoparticles mixed with 100 μg of plasmid DNA encoding GFP (n=3). One day after injection, popliteal lymph nodes were removed and cell suspensions were stained with monoclonal antibody against MHC Class II conjugated with PE. Cells were analyzed by flow cytometry. Histograms show the percentage of MHC Class II positive cells with GFP signal. Each histogram represents an individual mouse.

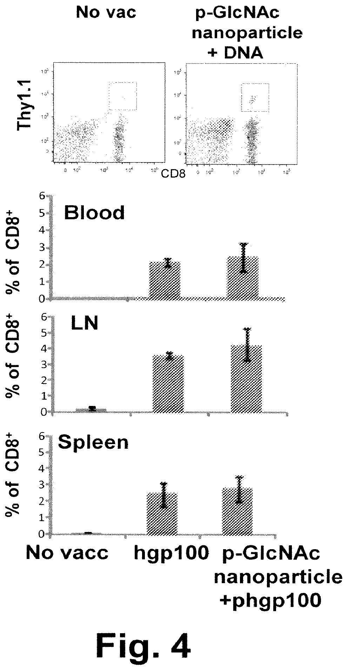

Figure 4. Proliferation of donor Pmel cells in response to hgp100 DNA vaccination. p-GlcNAc nanoparticle/phgp100 vaccination induces CD8+ specific responses. Mice were subcutaneously vaccinated as indicated 24 hours after adoptive transfer of 10$^6$ Pmel splenocytes (Thy1.1$^+$). Levels of circulating Pmel cells were determined by flow cytometry. Frequency of donor cells is shown as average of percentage of total CD8$^+$ T cells (n=3)±SDEV.

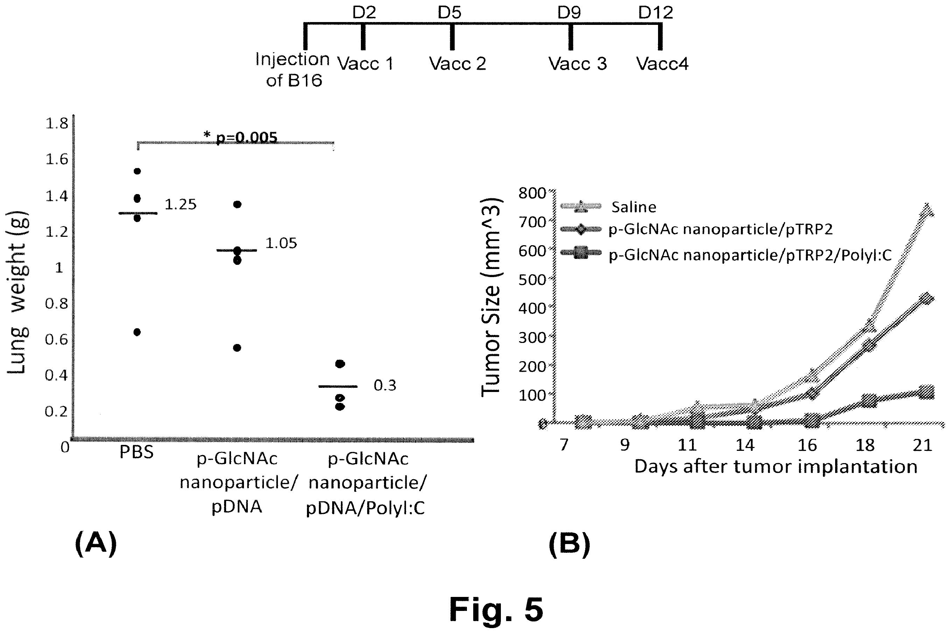

Figure 5. Co-delivery of DNA and Poly I:C with p-GlcNAc nanoparticles enhances antitumor immunity and the therapeutic efficacy of DNA vaccines encoding self tumor antigens. (A) Mice (n=5) were injected intravenously (i.v.) with 3x10$^4$ B16 melanoma cells. Three subcutaneous vaccinations were given three days apart starting at day three after B16 tumor cell injection (i.e., with saline control, p-GlcNAc nanoparticle/pTRP2, or p-GlcNAc nanoparticle/pTRP2/Poly I:C). Subsequently, animals were sacrificed, and their lungs were excised and weighed. (B) Mice (n=5) were injected subcutaneously (s.c.) with 10$^5$ B16 melanoma cells. Three subcutaneous vaccinations were given three days apart starting at day five after B16 tumor cell injection (i.e., with saline control, p-GlcNAc nanoparticle/pTRP2, or p-GlcNAc nanoparticle/pTRP2/Poly I:C). Following treatment, tumor progression was monitored three times a week.

## 5. DETAILED DESCRIPTION

### 5.1 p-GlcNAc Nanoparticle/ Nucleic Acid Compositions

Described herein are p-GlcNAc nanoparticle/nucleic acid compositions. In certain embodiments, p-GlcNAc nanoparticle/DNA compositions comprise poly-N-acetylglucosamine or a derivative thereof. In some embodiments, the poly-N-acetylglucosamine has a β-1→4 configuration. In other embodiments, the poly-N-acetylglucosamine has a α-1→4 configuration. In certain embodiments, the poly-N-acetylglucosamine is about 100%, 99.9%, 99.8%, 99.5%, 99%, 98%, 95%, 90%, 80%, 70%, 60%, 50%, 40%, 30%, or 20% pure. In a specific embodiment, the poly-N-acetylglucosamine is 90 to 100% pure. In some embodiments, the poly-N-acetylglucosamine is more than 90%, more than 95%, more than 98%, more than 99% pure, or more than 99.5% pure. In certain embodiments, 40% to 80%, 40% to 65%, 50% to 65%, 50% to 80%, 60% to 80%, 65% to 75%, 65% to 80% or 75% to 80%, of the poly-N-acetylglucosamine is deacetylated. In certain examples 25% to 50%, 40% to 95%, 40% to 90%, 50% to 95%, 50% to 90%, 60% to 95%, 60% to 90%, 65% to 95%, 65% to 90%, 65% to 80%, 70% to 90%, 75% to 85%, 85% to 95%, 90% to 99% or 95% to 100% of the poly-N-acetylglucosamine is deacetylated. In some examples, 25%, 35%, 85%, 90%, 95%, 98%, 99% or 100% of the poly-N-acetylglucosamine is deacetylated. In some examples, at least or more than 25%, 35%, 40%, 45%, 50%, 55%, 60%, 65%, 70%, 75%, 80%, 85%, 90%, 95%, 98%, or 99% of the poly-N-acetylglucosamine is deacetylated. In specific embodiments, the poly-N-acetylglucosamine or deacetylated poly-N-acetylglucosamine is derivatized with an organic acid or mineral acid to form an ammonium salt in order to facilitate its solubilization. In certain embodiments, the poly-N-acetylglucosamine or deacetylated poly-N-acetylglucosamine is derivatized with lactic acid. In certain embodiments, the poly-N-acetylglucosamine or deacetylated poly-N-acetylglucosamine is derivatized with lactic acid to facilitate its solubilization. U.S. Patent Nos. 5,622,834; 5,623,064; 5,624,679; 5,686,115; 5,858,350; 6,599,720; 6,686,342; and 7,115,588 describe the poly-N-acetylglucosamine and derivatives thereof, and methods of producing the same.

Poly-N-acetylglucosamine can, for example, be produced by, and may be purified from, microalgae, preferably diatoms. The diatoms which may be used as starting sources for the production of the poly-N-acetylglucosamine include, but are not limited to members of the *Coscinodiscus* genus, the *Cyclotella* genus, and the *Thalassiosira* genus. Poly-N-acetylglucosamine may be obtained from diatom cultures via a number of different methods, including the mechanical force method and chemical/biological method known in the art (see, e.g., U.S. Patent Nos. 5,622,834; 5,623,064; 5,624,679; 5,686,115; 5,858,350; 6,599,720; 6,686,342; and 7,115,588. In certain embodiments, the poly-N-acetylglucosamine is not derived from one or more of the following: a shell fish, a crustacean, insect, fungi or yeasts. In certain embodiments, the compositions do not comprise collagen fibers. In certain embodiments, the poly-N-acetylglucosamine is about 100%, 99.9%, 99.8%, 99.5%, 99%, 98%, 95%, 90%, 80%, 70%, 60%, 50%, 40%, 30%, or 20% pure. In a specific embodiment, the poly-N-acetylglucosamine is 90 to 100% pure. In certain embodiments, the poly-N-acetylglucosamine are fibers of greater than 15 μm. In specific embodiments, the poly-N-acetylglucosamine fibers are greater than 15 μm in length. In some embodiment, more than 50%, more than 75%, more than 90%, more than 95%, more than 99% of the poly-N-acetylglucosamine fibers are greater than 15 μm in length. In one embodiment, 100% of the poly-N-acetylglucosamine fibers are greater than 15 μm in length. In some embodiments, the poly-N-acetylglucosamine are fibers of 50 to 200 μm, 50 to 150 μm, 50 to 100 μm, or 80 to 100 μm in length. In some embodiments, the poly-N-acetylglucosamine are fibers of 10 to 25 nm, 10 to 50nm, 25 to 50 nm, or 50 to 100 nm in diameter.

In some examples, the poly-N-acetylglucosamine are fibers of 1 to 5 nm, or 2 to 4 nm, or 2 to 10 nm in diameter.

The poly-N-acetylglucosamine can be deacetylated by treatment of poly-N-acetylglucosamine with a base to yield glucosamines residues with free amino groups. This hydrolysis process may be carried out with solutions of concentrated sodium hydroxide or potassium hydroxide at elevated temperatures. Alternatively, an enzymatic procedure utilizing a chitin deacetylase enzyme may be used for poly-N-acetylglucosamine deacylation using techniques known in the art. In a specific embodiment, the poly-N-acetylglucosamine is deacetylated using the methods described in Section 6.1, *infra.* In certain embodiments, the deacetylated poly-N-acetylglucosamine has a molecular weight of $1 \times 10^4$ Da to $3.5 \times 10^6$ Da, $5 \times 10^4$ Da to $3.5 \times 10^6$ Da, $1 \times 10^5$ Da to $3.5 \times 10^6$ Da, $5 \times 10^5$ Da to $3.5 \times 10^6$ Da, $1 \times 10^6$ Da to $3 \times 10^6$ Da, $1.5 \times 10^6$ Da to $3.5 \times 10^6$ Da, $1.5 \times 10^6$ Da to $3 \times 10^6$ Da, $2 \times 10^6$ Da to $3 \times 10^6$ Da, $2 \times 10^6$ Da to $5 \times 10^6$ Da, $2 \times 10^6$ Da to $8 \times 10^6$ Da. In a specific embodiment, the deacetylated poly-N-acetylglucosamine has a molecular weight of $2.8 \times 10^6$ Da. In some embodiments, the deacetylated poly-N-acetylglucosamine has a molecular weight of at least $1 \times 10^4$ Da. In one embodiment, the deacetylated poly-N-acetylglucosamine has a molecular weight of at least $2 \times 10^6$ Da. In some embodiments, the deacetylated poly-N-acetylglucosamine has a molecular weight of less than $3 \times 10^6$ Da.

In certain embodiments, the deacetylated poly-N-acetylglucosamine can be derivatized, including counterion substitution to form salt derivatives, with any organic acid or mineral acid to form a p-GlcNAc ammonium salt. In some embodiments, the organic acid has the structure RCOOH, where R is optionally substituted alkyl, alkenyl, or alkynyl. In some embodiments, R is optionally substituted alkyl. In some embodiments, R is alkyl substituted with one or more hydroxyl groups. In certain embodiments, RCOOH is glycolic acid or lactic acid. In other embodiments, RCOOH is citric, succinic, gluconic, glucoronic, malic, pyruvic, tartaric, tartronic or fumaric acid. In a particular embodiment, RCOOH is lactic acid. In certain embodiments, the ratio of deacetylated poly-N-acetylglucosamine to poly-N-acetylglucosamine is 1:1, 1.2: 2:1, 1:3, or 3:1. In a specific embodiment, the deacetylated poly-N-acetylglucosamine can be derivatized with lactic acid using the methods described in Section 6.1, *infra.* In some embodiments, the deacetylated poly-N-acetylglucosamine is derivatized to make it soluble. In certain embodiments, the deacetylated poly-N-acetylglucosamine is solubilized by incubation with any organic acid or mineral acid (described herein or known in the art). In specific embodiments, the deacetylated poly-N-acetylglucosamine is derivatized to form a soluble p-GlcNAc ammonium salt. In some embodiments, solubility of the deacetylated poly-N-acetylglucosamine is achieved at a pH of about 4 to a pH of about 5, e.g., pH 4, pH 4.5, pH 5 or a pH between 4 and 5. In one embodiment, the deacetylated poly-N-acetylglucosamine is incubated with lactic acid to make it soluble (for example, at pH 4 to pH 5 such as pH 4.5). In such embodiment, H+ ion is substituted by lactate counterion to facilitate solubilization of the deacetylated poly-N-acetylglucosamine.

In certain embodiments, p-GlcNAc nanoparticle/nucleic acid compositions comprise a deacetylated poly-N-acetylglucosamine ammonium salt derivative, such as a lactate derivative. In a specific embodiment, p-GlcNAc nanoparticle/nucleic acid compositions comprise a deacetylated poly-$\beta$-1→4-N-acetylglucosamine lactate derivative.

In certain embodiments, 40% to 80%, 50% to 80%, 60% to 80%, 65% to 80%, 75% to 80%, 40% to 65%, 50% to 65%, or 65% to 75% of the poly-N-acetylglucosamine is deacetylated. In some embodiments, 40%, 45%, 50%, 55%, 60%, 65%, 70%, 75%, or 80% of the poly-N-acetylglucosamine is deacetylated.

In certain examples, 25% to 50%, 40% to 95%, 40% to 90%, 50% to 95%, 50% to 90%, 60% to 95%, 60% to 90%, 65% to 95%, 65% to 90%, 70% to 95%, 70% to 90%, 75% to 85%, 85% to 95%, 90% to 99% or 95% to 100% of the poly-N-acetylglucosamine is deacetylated. In some examples, 25%, 35%, 85%, 90%, 95%, 98%, 99% or 100% of the poly-N-acetylglucosamine is deacetylated. In some examples, at least or more than 25%, 35%, 40%, 45%, 50%, 55%, 60%, 65%, 70%, 75%, 80%, 85%, 90%, 95%, 98%, 99% of the poly-N-acetylglucosamine is deacetylated. In a specific embodiment, p-GlcNAc nanoparticle/nucleic acid compositions are the compositions that result from the process described in Section 6.1, *infra.*

In a specific embodiment, p-GlcNAc nanoparticle/nucleic acid compositions comprise a nucleic acid, such as described in Section 5.2, *infra.* In certain embodiments, a p-GlcNAc nanoparticle/nucleic acid composition comprises 0.1 $\mu$g to 2 mg, 0.2 $\mu$g to 1 mg, 0.5 $\mu$g to 500 $\mu$g, 1 $\mu$g to 750 $\mu$g, 1 $\mu$g to 500 $\mu$g 1 $\mu$g to 200 $\mu$g, 1 $\mu$g to 100 $\mu$g, 1 $\mu$g to 50 $\mu$g, 5 $\mu$g to 25 $\mu$g, 5 $\mu$g to 15 $\mu$g, 50 $\mu$g to 150 $\mu$g, 1 $\mu$g to 5 $\mu$g, 2 $\mu$g to 5 $\mu$g, 1 $\mu$g to 10 $\mu$g, 5 $\mu$g to 10 $\mu$g, 5 $\mu$g to 15 $\mu$g, 10 $\mu$g to 15 $\mu$g, 10 $\mu$g to 20 $\mu$g, 15 $\mu$g to 25 $\mu$g, 100 $\mu$g to 750 $\mu$g, 100 $\mu$g to 500 $\mu$g, 100 $\mu$g to 1 mg, or 500 $\mu$g to 750 $\mu$g of a nucleic acid. In some embodiments, a p-GlcNAc nanoparticle/nucleic acid composition comprises two, three or more different types of nucleic acids. In some embodiments, a p-GlcNAc nanoparticle/nucleic acid composition comprises two, three or more different nucleic acids that encode two, three or more different peptides, polypeptides, or proteins. In certain embodiments, a p-GlcNAc nanoparticle/nucleic acid composition comprises an adjuvant in addition to a nucleic acid.

In certain embodiments, p-GlcNAc nanoparticle/nucleic acid compositions do not comprise a significant amount of protein material. In certain embodiments, p-GlcNAc nanoparticle/nucleic acid compositions do not comprise any protein or peptide adjuvant. In other embodiments, p-GlcNAc nanoparticle/nucleic acid compositions comprise no greater than 0.1%, 0.5% or 1% by weight of protein material. In some embodiments, p-GlcNAc nanoparticle/nucleic acid compositions comprise no greater than 0.1%, 0.5%, 1% or 2% by weight of protein material as determined by any technique known in the art (such as Coomassie staining). In other embodiments, the protein content of a p-GlcNAc nanoparticle/nucleic acid composition is undetectable by Coomassie staining. In yet other embodiments, p-GlcNAc nanoparticle/nucleic acid compositions comprise a protein or peptide adjuvant.

In certain embodiments, 25% to 50%, 40% to 65%, 50% to 65%, 65% to 75%, 75% to 85%, 85% to 95%, 90% to 99% or 95% to 100% of the nanoparticles in a p-GlcNAc nanoparticle/nucleic acid composition are about 5 nm to about 500 nm, about 5 nm to about 300 nm, about 5 nm to about 150 nm, about 10 nm to about 500 nm, about 10 nm to about 300 nm, about 10 nm to about 150 nm, about 20 nm to about 500 nm, about 20 nm to about 300 nm, about 20 nm to about 150 nm, about 25 nm to about 500 nm, about 25 nm to about 300 nm, about 25 nm to about 150 nm, about 50 nm to about 100 nm, about 75 nm to about 100 nm, about 100 nm to about 125 nm, about 100 nm to about 150 nm, about 100 nm to about 200 nm, about 150 nm to about 200 nm, about 50 nm to about 150 nm, or about 50 nm to about 200 nm in size as measured by, *e.g.,* transmission electron microscopy or scanning electron microscopy. In some embodiments, 25% to 50%, 40% to 65%, 50% to 65%, 65% to 75%, 75% to 85%, 85% to 95%, 90% to 99% or 95% to 100% of the nanoparticles in a p-GlcNAc nanoparticle/nucleic acid composition are 5 nm to 500 nm, 5 nm to 300 nm, 5 nm to 150 nm, 10 nm to 500 nm, 10 nm to 300 nm, 10 nm to 150 nm, 20 nm to 500 nm, 20 nm to 300 nm, 20 nm to 150 nm, 25 nm to 500 nm, 25 nm to 300 nm, 25 nm to 150 nm, 50 nm to 100 nm, 75 nm to 100 nm, 100 nm to 125 nm, 100 nm to 150 nm, 100 nm to 200 nm, 150 nm to 200 nm, 50 nm to 150 nm, or 50 nm to 200 nm in size as measured by, *e.g.,* transmission electron microscopy or scanning electron microscopy.

In certain embodiments, 25%, 35%, 45%, 50%, 55%, 60%, 65%, 70%, 75%, 80%, 85%, 90%, 95%, 98%, 99% or 100% of the nanoparticles in a p-GlcNAc nanoparticle/nucleic acid composition are about 5 nm to about 500 nm, about 5 nm to about 300 nm, about 5 nm to about 150 nm, about 10 nm to about 500 nm, about 10 nm to about 300 nm, about 10 nm to about 150 nm, about 20 nm to about 500 nm, about 20 nm to about 300 nm, about 20 nm to about 150 nm, about 25 nm to about 500 nm, about 25 nm to about 300 nm, about 25 nm to about 150 nm, about 50 nm to about 100 nm, about 75 nm to about 100 nm, about 100 nm to about 125 nm, about 100 nm to about 150 nm, about 100 nm to about 200 nm, about 150 nm to about 200 nm, about 50 nm to about 150 nm, or about 50 nm to about 200 nm in size as measured by, *e.g.,* transmission electron microscopy or scanning electron microscopy. In some embodiments, 25%, 35%, 45%, 50%, 55%, 60%, 65%, 70%, 75%, 80%, 85%, 90%, 95%, 98%, 99% or 100% of the nanoparticles in a p-GlcNAc nanoparticle/nucleic acid composition are 5 nm to 500 nm, 5 nm to 300 nm, 5 nm to 150 nm, 10 nm to 500 nm, 10 nm to 300 nm, 10 nm to 150 nm, 20 nm to 500 nm, 20 nm to 300 nm, 20 nm to 150 nm, 25 nm to 500 nm, 25 nm to 300 nm, 25 nm to 150 nm, 50 nm to 100 nm, 75 nm to 100 nm, 100 nm to 125 nm, 100 nm to 150 nm, 100 nm to 200 nm, 150 nm to 200 nm, 50 nm to 150 nm, or 50 nm to 200 nm in size as measured by, *e.g.,* transmission electron microscopy or scanning electron microscopy.

In certain embodiments, at least or more than 25%, 35%, 45%, 50%, 55%, 60%, 65%, 70%, 75%, 80%, 85%, 90%, 95%, 98%, or 99% of the nanoparticles in a p-GlcNAc nanoparticle/nucleic acid composition are about 5 nm to about 500 nm, about 5 nm to about 300 nm, about 5 nm to about 150 nm, about 10 nm to about 500 nm, about 10 nm to about 300 nm, about 10 nm to about 150 nm, about 20 nm to about 500 nm, about 20 nm to about 300 nm, about 20 nm to about 150 nm, about 25 nm to about 500 nm, about 25 nm to about 300 nm, about 25 nm to about 150 nm, about 50 nm to about 100 nm, about 75 nm to about 100 nm, about 100 nm to about 125 nm, about 100 nm to about 150 nm, about 100 nm to about 200 nm, about 150 nm to about 200 nm, about 50 nm to about 150 nm, or about 50 nm to about 200 nm in size as measured by, *e.g.,* transmission electron microscopy or scanning electron microscopy. In some embodiments, at least or more than 25%, 35%, 45%, 50%, 55%, 60%, 65%, 70%, 75%, 80%, 85%, 90%, 95%, 98%, or 99% of the nanoparticles in a p-GlcNAc nanoparticle/nucleic acid composition are 5 nm to 500 nm, 5 nm to 300 nm, 5 nm to 150 nm, 10 nm to 500 nm, 10 nm to 300 nm, 10 nm to 150 nm, 20 nm to 500 nm, 20 nm to 300 nm, 20 nm to 150 nm, 25 nm to 500 nm, 25 nm to 300 nm, 25 nm to 150 nm, 50 nm to 100 nm, 75 nm to 100 nm, 100 nm to 125 nm, 100 nm to 150 nm, 100 nm to 200 nm, 150 nm to 200 nm, 50 nm to 150 nm, or 50 nm to 200 nm in size as measured by, *e.g.,* transmission electron microscopy or scanning electron microscopy.

In certain embodiments, at least or more than 25%, 35%, 45%, 50%, 55%, 60%, 65%, 70%, 75%, 80%, 85%, 90%, 95%, 98%, or 99% of the nanoparticles in a p-GlcNAc nanoparticle/nucleic acid composition are about 50 nm to about 500 nm, about 50 nm to about 400 nm, about 50 to about 300 nm, about 50 nm to about 200 nm, about 75 nm to about 500 nm, about 75 nm to about 300 nm, about 100 nm to about 500 nm, about 100 nm to about 400 nm, about 100 nm to about 300 nm, about 150 nm to about 500 nm, about 150 nm to about 400 nm, or about 150 nm to about 300 nm in size as measured by, *e.g.,* transmission electron microscopy or scanning electron microscopy. In some embodiments, at least or more than 25%, 35%, 45%, 50%, 55%, 60%, 65%, 70%, 75%, 80%, 85%, 90%, 95%, 98%, or 99% of the nanoparticles in a p-GlcNAc nanoparticle/nucleic acid composition are 50 nm to 500 nm, 50 nm to 400 nm, 50 to 300 nm, 50 nm to about 200 nm, 75 nm to 500 nm, 75 nm to 300 nm, 100 nm to 500 nm, 100 nm to 400 nm, 100 nm to 300 nm, 150 nm to 500 nm, 150 nm to 400 nm, or 150 nm to 300 nm in size as measured by, *e.g.,* transmission electron microscopy or scanning electron microscopy.

In certain examples, at least or more than 25%, 35%, 45%, 50%, 55%, 60%, 65%, 70%, 75%, 80%, 85%, 90%, 95%, 98%, or 99% of the nanoparticles in a p-GlcNAc nanoparticle/nucleic acid composition are about 10 nm to about 800 nm, about 10 nm to about 600 nm, 50 nm to about 800 nm, or about 50 nm to about 600 nm in size as measured by, e.g., transmission electron microscopy or scanning electron microscopy. In some examples, at least or more than 25%, 35%, 45%, 50%, 55%, 60%, 65%, 70%, 75%, 80%, 85%, 90%, 95%, 98%, or 99% of the nanoparticles in a p-GlcNAc nanoparticle/nucleic acid composition are 10 nm to 800 nm, 10 nm to 600 nm, 50 nm to 800 nm, 50 nm to 600 nm in size as measured by, e.g., transmission electron microscopy or scanning electron microscopy.

The terms "about" and "approximately," when used herein to a modify numeric value or numeric range, indicate that reasonable deviations from the value or range, typically 10% above and 10% below the value or range, remain within the intended meaning of the recited value or range.

In some embodiments, the described sizes of the nanoparticles indicate the diameter of spherical nanoparticles. In certain embodiments, the described sizes indicate the length of one of the cross-sectional dimensions of a nanoparticle (e.g., the longest of the two cross-sectional dimensions).

In certain examples, at least 25%, at least 35%, at least 45%, at least 50%, at least 55%, at least 60%, at least 65%, at least 70%, at least 75%, at least 80%, at least 85%, at least 90%, at least 95%, at least 98%, or at least 99% of the nanoparticles in a p-GlcNAc nanoparticle/nucleic acid composition are less than 500 nm. In some examples, at least 25%, at least 35%, at least 45%, at least 50%, at least 55%, at least 60%, at least 65%, at least 70%, at least 75%, at least 80%, at least 85%, at least 90%, at least 95%, at least 98%, or at least 99% of the nanoparticles in a p-GlcNAc nanoparticle/nucleic acid composition are less than 300 nm. In certain examples, at least 25%, at least 35%, at least 45%, at least 50%, at least 55%, at least 60%, at least 65%, at least 70%, at least 75%, at least 80%, at least 85%, at least 90%, at least 95%, at least 98%, or at least 99% of the nanoparticles in a p-GlcNAc nanoparticle/nucleic acid composition are less than 250 nm. In some examples, at least 25%, at least 35%, at least 45%, at least 50%, at least 55%, at least 60%, at least 65%, at least 70%, at least 75%, at least 80%, at least 85%, at least 90%, at least 95%, at least 98%, or at least 99% of the nanoparticles in a p-GlcNAc nanoparticle/nucleic acid composition are less than 200 nm. In some of these examples, at least 25%, at least 35%, at least 45%, at least 50%, at least 55%, at least 60%, at least 65%, at least 70%, at least 75%, at least 80%, at least 85%, at least 90%, at least 95%, at least 98%, at least 99%, or 100% of the nanoparticles in a p-GlcNAc nanoparticle/nucleic acid composition are at least 5 nm, at least 10 nm, at least 25 nm, or at least 50 nm in size.

In certain examples, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 98%, or at least 99% of the nanoparticles are at least or greater than 5 nm, 10 nm, 20 nm, 25 nm, or 50 nm in size. In certain examples, more than 25%, more than 35%, more than 45%, more than 50%, more than 55%, more than 60%, more than 65%, more than 70%, more than 75%, more than 80%, more than 85%, more than 90%, more than 95%, more than 98%, or more than 99% of the nanoparticles are at least or greater than 5 nm, 10 nm, 20 nm, 25 nm, or 50 nm in size. In specific examples, 100% of the nanoparticles are at least or greater than 5 nm, 10 nm, 20 nm, 25 nm, or 50 nm in size. In some of these examples, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 98%, at least 99%, or 100% of the nanoparticles in a p-GlcNAc nanoparticle/nucleic acid composition are less than 100 nm, 500 nm, or 750 nm in size. In certain examples, 25% to 50%, 40% to 65%, 50% to 65%, 65% to 75%, 75% to 85%, 85% to 95%, 90% to 99% or 95% to 100% of the nanoparticles in a p-GlcNAc nanoparticle/nucleic acid composition are no greater than 800 nm in size as measured by, *e.g.*, transmission electron microscopy or scanning electron microscopy. In some examples, 25% to 50%, 40% to 65%, 50% to 65%, 65% to 75%, 75% to 85%, 85% to 95%, 90% to 99% or 95% to 100% of the nanoparticles in a p-GlcNAc nanoparticle/nucleic acid composition are no greater than 800 nm in size as measured by, *e.g.,* transmission electron microscopy or scanning electron microscopy. In some of these examples, at least 25%, at least 35%, at least 45%, at least 50%, at least 55%, at least 60%, at least 65%, at least 70%, at least 75%, at least 80%, at least 85%, at least 90%, at least 95%, at least 98%, at least 99%, or 100% of the nanoparticles in a p-GlcNAc nanoparticle/nucleic acid composition are at least 5 nm, 10 nm, 20 nm, 25 nm, or 50 nm in size.

In certain examples, 25% to 50%, 40% to 65%, 50% to 65%, 65% to 75%, 75% to 85%, 85% to 95%, 90% to 99% or 95% to 100% of the nanoparticles in a p-GlcNAc nanoparticle/nucleic acid composition are no greater than 600 nm in size as measured by, *e.g.*, transmission electron microscopy or scanning electron microscopy. In some examples, 25% to 50%, 40% to 65%, 50% to 65%, 65% to 75%, 75% to 85%, 85% to 95%, 90% to 99% or 95% to 100% of the nanoparticles in a p-GlcNAc nanoparticle/nucleic acid composition are no greater than 600 nm in size as measured by, *e.g.,* transmission electron microscopy or scanning electron microscopy. In some of these examples, at least 25%, at least 35%, at least 45%, at least 50%, at least 55%, at least 60%, at least 65%, at least 70%, at least 75%, at least 80%, at least 85%, at least 90%, at least 95%, at least 98%, at least 99%, or 100% of the nanoparticles in a p-GlcNAc nanoparticle/nucleic acid composition are at least 5 nm, 10 nm, 20 nm, 25 nm, or 50 nm in size.

In certain examples, 25% to 50%, 40% to 65%, 50% to 65%, 65% to 75%, 75% to 85%, 85% to 95%, 90% to 99% or 95% to 100% of the nanoparticles in a p-GlcNAc nanoparticle/nucleic acid composition are no greater than 500 nm in size as measured by, *e.g.*, transmission electron microscopy or scanning electron microscopy. In some examples, 25% to 50%, 40% to 65%, 50% to 65%, 65% to 75%, 75% to 85%, 85% to 95%, 90% to 99% or 95% to 100% of the nanoparticles in a p-GlcNAc nanoparticle/nucleic acid composition are no greater than 500 nm in size as measured by, *e.g.,* transmission electron microscopy or scanning electron microscopy. In some of these examples, at least 25%, at least 35%, at least 45%, at least 50%, at least 55%, at least 60%, at least 65%, at least 70%, at least 75%, at least 80%, at least 85%, at least 90%, at least 95%, at least 98%, at least 99%, or 100% of the nanoparticles in a p-GlcNAc nanoparticle/nucleic acid composition are at least 5 nm, 10 nm, 20 nm, 25 nm, or 50 nm in size.

In certain examples, 25% to 50%, 40% to 65%, 50% to 65%, 65% to 75%, 75% to 85%, 85% to 95%, 90% to 99% or 95% to 100% of the nanoparticles in a p-GlcNAc nanoparticle/nucleic acid composition are no greater than 400 nm in size as measured by, *e.g.,* transmission electron microscopy or scanning electron microscopy. In some examples, 25% to 50%, 40% to 65%, 50% to 65%, 65% to 75%, 75% to 85%, 85% to 95%, 90% to 99% or 95% to 100% of the nanoparticles in a p-GlcNAc nanoparticle/nucleic acid composition are no greater than 400 nm in size as measured by, *e.g.,* transmission electron microscopy or scanning electron microscopy. In some of these examples, at least 25%, at least 35%, at least 45%, at least 50%, at least 55%, at least 60%, at least 65%, at least 70%, at least 75%, at least 80%, at least 85%, at least 90%, at least 95%, at least 98%, at least 99%, or 100% of the nanoparticles in a p-GlcNAc nanoparticle/nucleic acid composition are at least 5 nm, 10 nm, 20 nm, 25 nm, or 50 nm in size.

In certain examples, 25% to 50%, 40% to 65%, 50% to 65%, 65% to 75%, 75% to 85%, 85% to 95%, 90% to 99% or 95% to 100% of the nanoparticles in a p-GlcNAc nanoparticle/nucleic acid composition are no greater than 300 nm in size as measured by, *e.g.,* transmission electron microscopy or scanning electron microscopy. In some examples, 25% to 50%, 40% to 65%, 50% to 65%, 65% to 75%, 75% to 85%, 85% to 95%, 90% to 99% or 95% to 100% of the nanoparticles in a p-GlcNAc nanoparticle/nucleic acid composition are no greater than 300 nm in size as measured by, *e.g.,* transmission electron microscopy or scanning electron microscopy. In some of these examples, at least 25%, at least 35%, at least 45%, at least 50%, at least 55%, at least 60%, at least 65%, at least 70%, at least 75%, at least 80%, at least 85%, at least 90%, at least 95%, at least 98%, at least 99%, or 100% of the nanoparticles in a p-GlcNAc nanoparticle/nucleic acid composition are at least 5 nm, 10 nm, 20 nm, 25 nm, or 50 nm in size.

In certain examples, 25% to 50%, 40% to 65%, 50% to 65%, 65% to 75%, 75% to 85%, 85% to 95%, 90% to 99% or 95% to 100% of the nanoparticles in a p-GlcNAc nanoparticle/nucleic acid composition are no greater than 200 nm in size as measured by, *e.g.,* transmission electron microscopy or scanning electron microscopy. In some examples, 25% to 50%, 40% to 65%, 50% to 65%, 65% to 75%, 75% to 85%, 85% to 95%, 90% to 99% or 95% to 100% of the nanoparticles in a p-GlcNAc nanoparticle/nucleic acid composition are no greater than 200 nm in size as measured by, *e.g.,* transmission electron microscopy or scanning electron microscopy. In some of these examples, at least 25%, at least 35%, at least 45%, at least 50%, at least 55%, at least 60%, at least 65%, at least 70%, at least 75%, at least 80%, at least 85%, at least 90%, at least 95%, at least 98%, at least 99%, or 100% of the nanoparticles in a p-GlcNAc nanoparticle/nucleic acid composition are at least 5 nm, 10 nm, 20 nm, 25 nm, or 50 nm in size.

In certain embodiments, the nanoparticles in a p-GlcNAc nanoparticle/nucleic acid composition have irregular geometry. In other embodiments, the nanoparticles in a p-GlcNAc nanoparticle/nucleic acid composition have regular geometric shapes (e.g., a round or spherical shape).

In a specific embodiment, a p-GlcNAc nanoparticle/nucleic acid composition is biocompatible and/or biodegradable. Biocompatibility may be determined by a variety of techniques, including, but not limited to such procedures as the elution test, intramuscular implantation, or intracutaneous or systemic injection into animal subjects. Such tests are described in U.S. Patent No. 6,686,342. In one embodiment, a p-GlcNAc nanoparticle/nucleic acid composition has an elution test score of "0," an intramuscular implantation test score of "0," an intracutaneous injection test score of "0," and/or a weight gain as opposed to weight loss in response to a systemic injection. In one embodiment, the polymer or fiber has an elution test score of "0."

In a specific embodiment, biodegradable p-GlcNAc nanoparticle/nucleic acid compositions degrade within about 1 day, 2 day, 5 day, 8 day, 12 day, 17 day, 25 day, 30 day, 35 day, 40 day, 45 day, 50 day, 55 day, 60 day, 65 day, 70 day, 75 day, 80 day, 85 day, 90 day, 95 day, or 100 days after administration or implantation into a patient. In one aspect, the slow biodegradable nature of the p-GlcNAc nanoparticle/nucleic acid compositions allows for sustained release of the nucleic acid. This property increases the efficiency of transfection of nucleic acid and protects the nucleic acid from degradation by the serum nucleases.

In certain aspects, a p-GlcNAc nanoparticle/nucleic acid composition is immunoneutral, in that it does not elicit an immune response. In specific aspects, a p-GlcNAc nanoparticle/nucleic acid composition is immunoneutral, in that it does not elicit an immune response when administered to an animal (e.g., injected subcutaneously or intramuscularly into an animal such as a mouse or a rabbit). The non-immunogenic nature of the p-GlcNAc nanoparticle/nucleic acid composition allows its repeated administration into a subject.

In some embodiments, p-GlcNAc nanoparticle/nucleic acid compositions have no biological reactivity as shown by one or more biocompatibility tests. In one embodiment, the p-GlcNAc nanoparticle/nucleic acid compositions have no biological reactivity as shown by an elution test, subcutaneous injection test, intramuscular implantation test and/or systemic injection test.

In certain embodiments, a p-GlcNAc nanoparticle/nucleic acid composition can be stored at 20°C to 30°C or 20°C to 25°C for a certain period of time before use. In a specific embodiment, a p-GlcNAc nanoparticle/nucleic acid composition can be stored at room temperature for a certain period of time before use. In one embodiment, a p-GlcNAc nanoparticle/nucleic acid composition can be stored at 20°C to 30°C or 20°C to 25°C for about 30 minutes, 45 minutes, 1 hour, 1.5 hours, or 2 hours. In another embodiment, a p-GlcNAc nanoparticle/nucleic acid composition can be stored at 20°C to 30°C or 20°C to 25°C for 30 to 45 minutes, 45 minutes to 1 hour, 1 hour to 1.5 hours, 1 to 2 hours, or 1.5 to 2 hours. In one embodiment, a p-GlcNAc nanoparticle/nucleic acid composition can be stored at room temperature for about 30 minutes, 45 minutes, 1 hour, 1.5 hours, or 2 hours. In another embodiment, a p-GlcNAc nanoparticle/nucleic acid composition can be stored at room temperature for 30 to 45 minutes, 45 minutes to 1 hour, 1 hour to 1.5 hours, 1 to 2 hours, or 1.5 to 2 hours. In specific embodiments, a p-GlcNAc nanoparticle/nucleic acid composition can be stored at 4°C, 20°C to 30°C, 20°C to 25°C or at room temperature for up to about 30 minutes, 45 minutes, 1 hour, 1.5 hours, or 2 hours. In some embodiments a p-GlcNAc nanoparticle/nucleic acid composition can be stored at 4°C, 20°C to 30°C, 20°C to 25°C or at room temperature for more than 2 hours (e.g., 3 hours, 4 hours, 5 hours, 6 hours, 7 hours, 8 hours, 12 hours, or 24 hours), or for more than 1 day. In one embodiment, a p-GlcNAc nanoparticle/nucleic acid composition is stored at 4°C. In a specific embodiment, a p-GlcNAc nanoparticle/nucleic acid composition can be frozen or cryopreserved (and thawed before administration to a patient). For example, a p-GlcNAc nanoparticle/nucleic acid composition can be frozen at -20°C or -70°C. In other embodiments, a p-GlcNAc nanoparticle/nucleic acid composition is not frozen, or not cryopreserved and thawed, prior to administration to a patient.

### 5.2 Nucleic Acids

A p-GlcNAc nanoparticle/nucleic acid composition can comprise any nucleic acid known to one skilled in the art. Such nucleic acids include, but are not limited to, DNA and RNA, including cDNA, genomic DNA, plasmid DNA, plasmid RNA, mRNA, siRNA, microRNA, single stranded RNA, double stranded RNA, oligonucleotides, single stranded or double stranded oligonucleotides, triplex oligonucleotides, and other nucleic acids. Nucleic acids encompassed herein include nucleic acids in a sense or antisense orientations, modified, unmodified and synthetic nucleic acids. In specific embodiments, the nucleic acid is a coding region of a gene.

In one aspect, a p-GlcNAc nanoparticle/nucleic acid composition comprises a nucleic acid encoding a therapeutic peptide, polypeptide or protein. Such a therapeutic peptide, polypeptide or protein may be useful in treatment and/or prevention of a disorder in which the production of the therapeutic peptide, polypeptide or protein is beneficial to a subject, such as cancer, infectious diseases, genetic deficiencies of certain necessary proteins, and/or acquired metabolic or regulatory imbalances. For example, nucleic acid encoding a cytokine, such as interferon, IL-2, IL-12 or IL-15 might be useful for the treatment and/or prevention of infectious diseases and/or cancer. Nucleic acids encoding a insulin like growth factor binding protein 7 (IGFBP-7) and other factors might be useful for reducing the proliferation of certain cancer cells (*e.g.*, breast cancer cells) and/or the growth of certain types of tumors (*e.g.,* breast tumors). Nucleic acids encoding insulin might be useful to treating and/or preventing diabetes. Nucleic acids encoding, *e.g.,* acid sphingomyelinase might be useful to treat Niemann-Pick disease.

In another aspect, a p-GlcNAc nanoparticle/nucleic acid composition comprises a nucleic acid encoding an antigen. The nucleic acid can encode any disease target of interest. For example, the nucleic acid can encode viral antigens, bacterial antigens, fungal antigens, parasitic antigens, and/or tumor-associated antigens. In a specific embodiment, the nucleic acid encodes a self-antigen. Nonlimiting examples of viral antigens include antigens from adenovirdiae (*e.g.*, mastadenovirus and aviadenovirus), herpesviridae (*e.g.*, herpes simplex virus 1, herpes simplex virus 2, herpes simplex virus 5, herpes simplex virus 6, Epstein-Barr virus, HHV6-HHV8 and cytomegalovirus), leviviridae (*e.g.*, levivirus, enterobacteria phase MS2, allolevirus), poxviridae (*e.g.*, chordopoxvirinae, parapoxvirus, avipoxvirus, capripoxvirus, leporipoxvirus, suipoxvirus, molluscipoxvirus, and entomopoxvirinae), papovaviridae (*e.g.*, polyomavirus and papillomavirus), paramyxoviridae (*e.g.,* paramyxovirus, parainfluenza virus 1, mobillivirus (*e.g.,* measles virus), rubulavirus (*e.g.,* mumps virus), pneumonovirinae (*e.g.,* pneumovirus, human respiratory synctial virus), human respiratory syncytial virus and metapneumovirus (*e.g.,* avian pneumovirus and human metapneumovirus)), picornaviridae (*e.g.*, enterovirus, rhinovirus, hepatovirus (*e.g.,* human hepatits A virus), cardiovirus, and apthovirus), reoviridae (*e.g.*, orthoreovirus, orbivirus, rotavirus, cypovirus, fijivirus, phytoreovirus, and oryzavirus), retroviridae (*e.g.*, mammalian type B retroviruses, mammalian type C retroviruses, avian type C retroviruses, type D retrovirus group, BLV-HTLV retroviruses, lentivirus (*e.g.* human immunodeficiency virus 1 and human immunodeficiency virus 2), spumavirus), flaviviridae (*e.g.,* hepatitis C virus), hepadnaviridae (*e.g.,* hepatitis B virus), togaviridae (*e.g.,* alphavirus (*e.g.,* sindbis virus) and rubivirus (*e.g.,* rubella virus)), rhabdoviridae (*e.g.,* vesiculovirus, lyssavirus, ephemerovirus, cytorhabdovirus, and necleorhabdovirus), arenaviridae (*e.g.*, arenavirus, lymphocytic choriomeningitis virus, Ippy virus, and lassa virus), and coronaviridae (*e.g.*, coronavirus and torovirus).

Nonlimiting examples of bacterial antigens include antigens from bacteria of the *Aquaspirillum* family, *Azospirillum* family, *Azotobacteraceae* family, *Bacteroidaceae* family, *Bartonella* species, *Bdellovibrio* family, *Campylobacter* species, *Chlamydia* species (*e.g., Chlamydia pneumoniae*), clostridium, *Enterobacteriaceae* family (*e.g., Citrobacter* species, Edwardsiella, *Enterobacter aerogenes, Erwinia* species, *Escherichia coli,* Hafnia species, *Klebsiella* species, Morganella species, *Proteus vulgaris,* Providencia, *Salmonella* species, *Serratia marcescens,* and *Shigella flexneri*), *Gardinella* family, *Haemophilus influenzae, Halobacteriaceae* family, *Helicobacter* family, *Legionallaceae* family, *Listeria* species, *Methylococcaceae* family, mycobacteria (*e.g., Mycobacterium tuberculosis*), *Neisseriaceae* family, *Oceanospirillum* family, *Pasteurellaceae* family, *Pneumococcus* species, *Pseudomonas* species, *Rhizobiaceae* family, *Spirillum* family, *Spirosomaceae* family, *Staphylococcus* (*e.g.*, methicillin resistant *Staphylococcus aureus* and *Staphylococcus pyrogenes*), *Streptococcus* (*e.g., Streptococcus enteritidis, Streptococcus fasciae,* and *Streptococcus pneumoniae*), *Vampirovibr Helicobacter* family, and *Vampirovibrio* family.

Nonlimiting examples of fungal antigens include antigens from fungus of *Absidia* species (e.g., *Absidia corymbifera* and *Absidia ramosa*), *Aspergillus* species, (e.g., *Aspergillus flavus, Aspergillus fumigatus, Aspergillus nidulans, Aspergillus niger,* and *Aspergillus terreus*), *Basidiobolus ranarum, Blastomyces dermatitidis, Candida* species (e.g., *Candida albicans, Candida glabrata, Candida kerr, Candida krusei, Candida parapsilosis, Candida pseudotropicalis, Candida quillermondii, Candida rugosa, Candida stellatoidea,* and *Candida tropicalis*), *Coccidioides immitis, Conidiobolus* species, *Cryptococcus* neoforms, *Cunninghamella* species, dermatophytes, *Histoplasma capsulatum, Microsporum gypseum, Mucor pusillus, Paracoccidioides brasiliensis, Pseudallescheria boydii, Rhinosporidium seeberi,* Pneumocystis carinii, *Rhizopus* species (e.g., *Rhizopus arrhizus, Rhizopus oryzae,* and *Rhizopus microsporus*), *Saccharomyces* species, *Sporothrix schenckii,* zygomycetes, and classes such as *Zygomycetes, Ascomycetes,* the *Basidiomycetes, Deuteromycetes,* and *Oomycetes.*

Non-limiting tumor-associated antigens include melanocyte lineage proteins (such as gp100, MART-1/MelanA, TRP-1 (gp75), and tyrosinase), and tumor-specific antigens (such as MAGE-1, MAGE-3, BAGE, GAGE-1, -2, N-acetylglucosaminyltransferase-V, p15, beta-catenin, MUM-1, CDK4, Nonmelanoma antigens, HER-2/neu (breast and ovarian carcinoma), Human papillomavirus-E6, E7 (cervical carcinoma), and MUC-1 (breast, ovarian and pancreatic carcinoma)).

Nucleic acid sequences encoding a therapeutic peptide, polypeptide or protein, or an antigen can be determined by cloning techniques or found within sequence databases such as, GenBank and Uniprot.

In certain embodiments, the nucleic acids described above may be part of or otherwise contained in a vector or plasmid that provides transcriptional regulatory elements and optionally, translational regulatory elements. The vector or plasmid chosen will depend upon a variety of factors, including, without limitation, the strength of the transcriptional regulatory elements.

Techniques for practicing aspects of this invention will employ, unless otherwise indicated, conventional techniques of molecular biology and recombinant DNA manipulation and production, which are routinely practiced by one of skill in the art.

**5.3 Adjuvants**

In certain embodiments, a p-GlcNAc nanoparticle/nucleic acid composition described herein comprises, or are administered in combination with, an adjuvant. The adjuvant for administration in combination with a composition described herein may be administered before, concomitantly with, or after administration of said composition. In specific embodiments, the adjuvant is administered in a p-GlcNAc nanoparticle/nucleic acid composition. In other embodiments, the adjuvant is administered concomitantly with but not in the same composition as the nucleic acid.

In some embodiments, the term "adjuvant" refers to a compound that when administered in conjunction with or as part of a composition described herein augments, enhances and/or boosts an immune response. For example, an adjuvant can enhance and/or boost an immune response to an influenza virus hemagglutinin, but when the compound is administered alone does not generate an immune response. In some embodiments, the adjuvant generates an immune response and does not produce an allergy or another adverse reaction. Adjuvants can enhance an immune response by several mechanisms including, *e.g.,* lymphocyte recruitment, stimulation of B and/or T cells, and stimulation of macrophages.

In certain embodiments, an adjuvant augments the intrinsic immune response to the antigen encoded by the nucleic acid in a p-GlcNAc nanoparticle/nucleic acid composition. In certain embodiments, an adjuvant augments the intrinsic immune response to the antigen encoded by the nucleic acid in a p-GlcNAc nanoparticle/nucleic acid composition without causing conformational changes in the product encoded by the nucleic acid. In certain embodiments, an adjuvant augments the intrinsic immune response to the antigen encoded by the nucleic acid in a p-GlcNAc nanoparticle/nucleic acid composition without causing conformational changes in the product encoded by the nucleic acid that affects the qualitative form of the response.

In specific embodiments, the adjuvant is a protein or a peptide. In other embodiments, the adjuvant is not a protein or peptide. In some embodiment, the adjuvant is a chemical. In other embodiments, the adjuvant is not a chemical.

In some embodiments, an adjuvant is a nucleic acid. Such adjuvant can be placed in the same or in a different construct from the "primary" nucleic acid to be delivered in a p-GlcNAc nanoparticle/nucleic acid composition. Such adjuvant can be added either to the same "primary" p-GlcNAc nanoparticle/nucleic acid composition, or administered concomitantly or sequentially with the "primary" p-GlcNAc nanoparticle/nucleic acid composition in a separate adjuvant/polymer vehicle. Two or more adjuvants (e.g., nucleic acid adjuvants) can be administered in two or more separate p-GlcNAc nanoparticle/nucleic acid compositions. In certain embodiments, the adjuvant is not a nucleic acid.

Specific examples of adjuvants include, but are not limited to, aluminum salts (alum) (such as aluminum hydroxide, aluminum phosphate, and aluminum sulfate), 3 De-O-acylated monophosphoryl lipid A (MPL) (see GB 2220211), MF59 (Novartis), AS03 (GlaxoSmithKline), AS04 (GlaxoSmithKline), polysorbate 80 (Tween 80; ICL Americas, Inc.), imidazopyridine compounds (see International Application No. PCT/US2007/064857, published as International Publication No. WO2007/109812), imidazoquinoxaline compounds (see International Application No. PCT/US2007/064858, published as International Publication No. WO2007/109813) and saponins, such as QS21 (see Kensil et al., in Vaccine Design: The Subunit and Adjuvant Approach (eds. Powell & Newman, Plenum Press, NY, 1995); U.S. Pat. No. 5,057,540). In some embodiments, the adjuvant is Freund's adjuvant (complete or incomplete). Other adjuvants are oil in water emulsions (such as squalene or peanut oil), optionally in combination with immune stimulants, such as monophosphoryl lipid A (see Stoute et al., N. Engl. J. Med. 336, 86-91 (1997)). Another adjuvant is CpG (Bioworld Today, Nov. 15, 1998). Such adjuvants can be used with or without other specific immunostimulating agents such as MPL or 3-DMP, QS21, polymeric or monomeric amino acids such as polyglutamic acid or polylysine, or other immunopotentiating agents known in the art.

In one aspect, an adjuvant is a cytokine, *e.g.,* GM-CSF, IL-2, IL-12, IL-15, TNF-α, and IFN-α. In another aspect, the adjuvant is polyinosinic:polycytidylic acid("Poly I:C") or CPG. In one embodiment, the adjuvant is Poly I:C. In some embodiments, the adjuvant is used at a concentration of about 1 μg to 100 μg per one dose of administration. In some embodiments, the adjuvant is used at a concentration of about 0.5 μg to 200 μg, 1 μg to 150 μg, 1 μg to 20 μg, 1 μg to 50 μg, 10 μg to 25 μg, 10 μg to 50 μg, 10 μg to 75 μg, 10 μg to 100 μg, 10 μg to 150 μg, 20 μg to 50 μg, 20 μg to 80 μg, 20 μg to 100 μg, 25 μg to 75 μg, 50 μg to 75 μg, 50 μg to 100 μg, or 50 μg to 150 μg per one dose of administration. In specific embodiments, Poly I:C or CpG is used at a concentration of about 1 μg to 500 μg, 10 μg to 250 μg, 20 μg to 200 μg, 25 μg to 150 μg, 25 μg to 100 μg, 25 μg to 75 μg, 30 μg to 70 μg, or 40 μg to 60 μg per one dose of administration. In other specific embodiments, GM-CSF or IL-12 is used at a concentration of about 0.1 μg to 250 μg, 0.5 μg to 100 μg, 0.5 μg to 75 μg, 0.5 μg to 50 μg, 1 μg to 100 μg, 1 μg to 50 μg, 1 μg to 25 μg, 1 μg to 15 μg, 1 μg to 10 μg, 2 μg to 15 μg, 2 μg to 10 μg, or 2.5 μg to 7.5 μg per one dose of administration. In a specific embodiment, an adjuvant is added to or used in combination with a p-GlcNAc nanoparticle/nucleic acid composition.

### 5.4 Methods of Making p-GlcNAc Nanoparticle/Nucleic Acid Compositions

In certain embodiments, p-GlcNAc compositions comprising deacetylated poly-N-acetylglucosamine derivatized with a mineral acid or an organic acid to allow it to be solubilized (as described *supra*, section 5.1), such as lactic, citric, succinic, gluconic, glucoronic, malic, pyruvic, tartaric, tartronic or fumaric acid, are diluted in a buffer (e.g., an acetic acid buffer such as sodium acetate-acetic acid buffer or ammonium acetate-acetic acid buffer), and optionally, incubated for a certain period of time (*e.g.,* 5 to 10 minutes, 5 to 15 minutes, 10 to 15 minutes, 10 to 20 minutes, 15 to 30 minutes, 30 to 45 minutes, 30 minutes to 1 hour, 45 minutes to 1 hour, 5 minutes to 1 hour, 10 minutes to 1 hour, or for at least for 5 or 10 minutes) at a certain temperature (*e.g.*, 45° C to 55° C, 50° C to 55° C, 50° C to 60° C, 55° C to 60° C, 55° C to 65° C, 60° C to 75° C, or 45° C to 75°). In certain embodiments, the organic acid has the structure RCOOH, where R is optionally substituted alkyl, alkenyl, or alkynyl. In some embodiments, R is optionally substituted alkyl. In some embodiments, R is alkyl substituted with one or more hydroxyl groups. In certain embodiments, RCOOH is glycolic acid or lactic acid. In other embodiments, RCOOH is citric, succinic, gluconic, glucoronic, malic, pyruvic, tartaric, tartronic or fumaric acid. In specific embodiments, the buffer used in the method described herein can be any buffer which is effective to dilute the p-GlcNAc compositions.

In some embodiments, p-GlcNAc compositions comprising deacetylated poly-N-acetylglucosamine derivatized/solubilized with a mineral acid or an organic acid to form an ammonium salt derivative (as described *supra*, section 5.1), such as lactic, citric, succinic, gluconic, glucoronic, malic, pyruvic, tartaric, tartronic or fumaric acid, are dissolved/diluted in a buffer, such as sodium acetate-acetic acid buffer pH 5.7 (or ammonium acetate-acetic acid buffer), and incubated for a certain period of time (*e.g.*, 5 to 10 minutes, 5 to 15 minutes, 10 to 15 minutes, 10 to 20 minutes, 15 to 30 minutes, 30 to 45 minutes, 30 minutes to 1 hour, or 45 minutes to 1 hour) at a certain temperature (*e.g.*, 45° C to 55° C, 50° C to 55° C, 50° C to 60° C, 55° C to 60° C, 55° C to 65° C, or 60° C to 75° C). In one embodiment, a p-GlcNAc composition comprising deacetylated poly-N-acetylglucosamine derivatized/solubilized with lactic acid is dissolved/diluted in buffer, such as sodium acetate-acetic acid buffer pH 5.7 (or ammonium acetate-acetic acid buffer), to obtain a final concentration of the derivatized poly-N-acetylglucosamine of about 0.001% to about 0.01%, about 0.01% to about 0.1%, about 0.1% to about 0.2%, about 0.1% to about 0.25%, about 0.1% to about 0.3%, about 0.1% to about 0.4%, about 0.1% to about 0.5%, about 0.1% to about 1%, about 0.2% to about 0.3%, about 0.2 to about 0.4% or about 0.2% to about 0.5%. In another embodiment, a p-GlcNAc composition comprising deacetylated poly-N-acetylglucosamine derivatized/solubilized with lactic acid is dissolved/diluted in a buffer, such as sodium acetate-acetic acid buffer pH 5.7 (or ammonium acetate-acetic acid buffer), to obtain a final concentration of the derivatized poly-N-acetylglucosamine of 0.001% to 0.01%, 0.01% to 0.1%, 0.1% to 0.2%, 0.1% to 0.25%, 0.1% to 0.3%, 0.1% to 0.4%, 0.1% to 0.5%, 0.1% to 1%, 0.2% to 0.3%, 0.2 to 0.4% or 0.2% to 0.5%. In a specific embodiment, a p-GlcNAc composition comprising deacetylated poly-N-acetylglucosamine derivatized/solubilized with lactic acid is dissolved/diluted in a buffer, such as sodium acetate-acetic acid buffer pH 5.7 (or ammonium acetate-acetic acid buffer), to obtain a final concentration of the derivatized poly-N-acetylglucosamine of 0.2%. In one embodiment, the buffer chosen precipitates the p-GlcNAc composition.

A certain amount of the dissolved/diluted p-GlcNAc composition can then be combined with a certain concentration of a nucleic acid and the mixture can be agitated (by, *e.g.*, mixing, vortexing or shaking) for a certain period of time (e.g., 5 to 10 seconds, 5 to 15 seconds, 5 to 20 seconds, 10 to 20 seconds, 20 to 30 seconds, 20 to 40 seconds, 30 to 40 seconds, 40 to 50 seconds, 50 to 60 seconds, 1 to 2 minutes, 2 to 4 minutes, or 2 to 5 minutes) to form p-GlcNAc nanoparticle/nucleic acid compositions described herein. In certain embodiments, 50 to 100 microliters, 75 to 150 microliters, 75 to 100 microliters, or 100 to 200 microliters of the dissolved/diluted p-GlcNAc composition is combined with a certain concentration of a nucleic acid. In a specific embodiment, 100 microliters of the dissolved/diluted p-GlcNAc composition is combined with a certain concentration of a nucleic acid. In certain embodiments, the nucleic acid has been combined with a salt, such as sodium sulfate, potassium sulfate, calcium sulfate or magnesium sulfate, and incubated at certain temperature (*e.g.*, 45° C to 55° C, 50° C to 55° C, 50° C to 60° C, 55° C to 60° C, 55° C to 65° C, or 60° C to 75° C) for a certain period of time (*e.g.*, 5 to 10 minutes, 5 to 15 minutes, 10 to 15 minutes, 10 to 20 minutes, 15 to 30 minutes, 30 to 45 minutes, 30 minutes to 1 hour, or 45 minutes to 1 hour). In a specific embodiment, 0.1 $\mu$g to 2 mg, 0.2 $\mu$g to 1 mg, 0.5 $\mu$g to 500 $\mu$g, 1 $\mu$g to 200 $\mu$g, 1 $\mu$g to 100 $\mu$g, 1 $\mu$g to 50 $\mu$g, 5 $\mu$g to 25 $\mu$g, 5 $\mu$g to 15 $\mu$g, 50 $\mu$g to 150 $\mu$g, 1 $\mu$g to 5 $\mu$g, 2 $\mu$g to 5 $\mu$g, 1 $\mu$g to 10 $\mu$g, 5 $\mu$g to 10 $\mu$g, 5 $\mu$g to 15 $\mu$g, 10 $\mu$g to 15 $\mu$g, 10 $\mu$g to 20 $\mu$g, or 15 $\mu$g to 25 $\mu$g of nucleic acid are combined with a salt, such as sodium sulfate, potassium sulfate, calcium sulfate or magnesium sulfate. In a specific embodiment, the nucleic acid is combined with 100 microliters of 50 mM sodium sulfate. In certain embodiments, the mixture of dissolved/diluted p-GlcNAc composition and nucleic acid is agitated by vortexing for a certain period of time (e.g., 5 to 10 seconds, 5 to 15 seconds, 5 to 20 seconds, 10 to 20 seconds, 20 to 30 seconds, 20 to 40 seconds, 30 to 40 seconds, 40 to 50 seconds, 50 to 60 seconds, 1 to 2 minutes, 2 to 4 minutes, or 2 to 5 minutes). In a specific embodiment, the mixture of dissolved/diluted p-GlcNAc composition and nucleic acid is agitated by vortexing for 20 seconds. In certain embodiments, an adjuvant as well as the nucleic acid is combined with the dissolved/diluted p-GlcNAc composition. See Section 5.3, *supra*, for examples of adjuvants that might be added to the p-GlcNAc nanoparticle/nucleic acid compositions.

A nucleic acid can be prepared for use in the method of making p-GlcNAc nanoparticle/nucleic acid composition by combining or mixing it with a salt, such as sodium sulfate, potassium sulfate, calcium sulfate or magnesium sulfate, and optionally, incubating the resulting combination or mixture at certain temperature (*e.g.*, 45° C to 55° C, 50° C to 55° C, 50° C to 60° C, 55° C to 60° C, 55° C to 65° C, 60° C to 75° C, or 45° C to 75° C) for a certain period of time (*e.g.*, 5 to 10 minutes, 5 to 15 minutes, 10 to 15 minutes, 10 to 20 minutes, 15 to 30 minutes, 30 to 45 minutes, 30 minutes to 1 hour, 45 minutes to 1 hour, 5 minutes to 1 hour, 10 minutes to 1 hour, or for at least 5 or 10 minutes). In a specific embodiment, 0.1 $\mu$g to 2 mg, 0.2 $\mu$g to 1 mg, 0.5 $\mu$g to 500 $\mu$g, 1 $\mu$g to 200 $\mu$g, 1 $\mu$g to 100 $\mu$g, 1 $\mu$g to 50 $\mu$g, 5 $\mu$g to 25 $\mu$g, 5 $\mu$g to 15 $\mu$g, 50 $\mu$g to 150 $\mu$g, 1 $\mu$g to 5 $\mu$g, 2 $\mu$g to 5 $\mu$g, 1 $\mu$g to 10 $\mu$g, 5 $\mu$g to 10 $\mu$g, 5 $\mu$g to 15 $\mu$g, 10 $\mu$g to 15 $\mu$g, 10 $\mu$g to 20 $\mu$g, or 15 $\mu$g to 25 $\mu$g of nucleic acid are combined with a salt, such as sodium sulfate. In a specific embodiment, the nucleic acid is combined with 100 microliters of 50 mM sodium sulfate. In specific embodiments, 0.5 mg/ml to 100 mg/ml, 1 mg/ml to 50 mg/ml, 1 mg/ml to 30 mg/ml, 1 mg/ml to 20 mg/ml, 2 mg/ml to 50 mg/ml, 2 mg/ml to 30 mg/ml, 2 mg/ml to 20 mg/ml, 3 mg/ml 30 mg/ml, 3 mg/ml to 20 mg/ml, 4 mg/ml to 15 mg/ml, 5 mg/ml to 15 mg/ml, 5 mg/ml to 10 mg/ml, or 6 mg/ml to 8 mg/ml of sodium sulfate is combined with a nucleic acid.

In a specific embodiment, the methodology described in Section 6.1, *infra*, is used to produce a p-GlcNAc nanoparticle/nucleic acid composition.

### 5.5 Uses of p-GlcNAc Nanoparticle/Nucleic Acid Compositions

Described herein are methods for *in vivo* and *ex vivo* delivery of a nucleic acid to a subject.
In the context of the invention, references to methods of treatment or methods of administration are to be understood as referring to the composition of the invention for use in said methods. Accordingly, aspects and embodiments relating to such methods are to be understood as those relating to compounds or compositions for use in the above sense.

In a specific embodiment, methods for delivery of a nucleic acid to a subject *in vivo* for the purposes of gene therapy or vaccination are contemplated. The methods generally comprise administering a p-GlcNAc nanoparticle/nucleic acid composition to a subject. In certain embodiments, the p-GlcNAc nanoparticle/nucleic acid composition comprises an adjuvant in addition to a nucleic acid. In other embodiments, an adjuvant is administered separately before, during or after the administration of a p-GlcNAc nanoparticle/nucleic acid composition.

In one embodiment, the administration of a p-GlcNAc nanoparticle/nucleic acid composition results in a sustained expression of a nucleic acid in the composition. In certain embodiments, the administration of a p-GlcNAc nanoparticle/nucleic acid composition results in expression of a nucleic acid in the composition for 1 week, 2 weeks, 3 weeks, 4 weeks, 1 month, 1.5 months, 2 months, 3 months, 4 months, 5 months, 6 months, 8 months, 10 months, 1 year or longer. In certain embodiments, the administration of a p-GlcNAc nanoparticle/nucleic acid composition results in expression of a nucleic acid for a period of time between 2 hours and 3 months, 2 hours and 2 months, 2 hours and 1 month, 2 hours and 2 weeks, 6 hours and 3 months, 6 hours and 2 months, 6 hours and 1 month, 6 hours and 2 weeks, 12 hours and 3 months, 12 hours and 2 months, 12 hours and 1 month, 12 hours and 2 weeks, 1 day and 3 months, 1 day and 2 months, 1 day and 1 month, 1 day and 2 weeks, 2 days and 3 months, 2 days and 2 months, 2 days and 1 month, or 2 days and 2 weeks post-administration.

In another embodiment, the administration of a p-GlcNAc nanoparticle/nucleic acid composition comprising an adjuvant is able to co-deliver nucleic acids and adjuvants to a subject. In a specific embodiment, the p-GlcNAc nanoparticle/nucleic acid composition is able to efficiently release adjuvants, such as GM-CSF and IL-12, for a sustained concurrent release of both nucleic acid and adjuvant. Without being bound by any theory, the co-delivery of nucleic acids and adjuvant by the p-GlcNAc nanoparticle/nucleic acid composition increases the likelihood that antigen-presenting cells uptake the nucleic acid under proper stimulatory conditions. This stimulatory condition will be useful, *e.g.*, when administering a nucleic acid encoding an antigen.

A p-GlcNAc nanoparticle/nucleic acid composition can be administered to a subject as part of a gene therapy protocol or vaccination protocol. The gene therapy or vaccination can be used to treat and/or prevent a variety of disorders or symptoms thereof. For example, gene therapy, can be used to treat and/or prevent cancer, infectious diseases, genetic deficiencies of certain necessary proteins, and/or acquired metabolic or regulatory imbalances.

A p-GlcNAc nanoparticle/nucleic acid composition can be administered to a subject by any route that permits expression of the nucleic acid, including parenteral, topical, intradermal, intranasal, mucosal intraperitoneal, epidural, sublingual, intracerebral, intravaginal, transdermal, rectal, by inhalation, intratumoral, and topical. Provided herein, a p-GlcNAc nanoparticle/nucleic acid composition is to be delivered to a subject subcutaneously, intramuscularly or intravenously. In a specific embodiment, a p-GlcNAc nanoparticle/nucleic acid composition is administered by subcutaneous injection. In certain cases, a p-GlcNAc nanoparticle/nucleic acid composition is not administered intravenously.

In a specific embodiment, a p-GlcNAc nanoparticle/nucleic acid composition is administered to the epithelial cells, *e.g.*, cells of the skin, epidermis or dermis. In one embodiment, a p-GlcNAc nanoparticle/nucleic acid composition is administered subcutaneously, e.g., by injection, in order to target the cells of the skin. The advantage of subcutaneous administrations is that such administration can target antigen presenting cells, such as dendritic cells, which play a central role in the initiation and establishment of a robust antigen-specific immune response. Delivery of the compositions described herein into the skin of a subject allows targeting of an antigen encoded by the nucleic acid to dendritic cells. In certain embodiments, a p-GlcNAc nanoparticle/nucleic acid composition is administered in combination with an adjuvant, *e.g.*, a cytokine. Subcutaneous administration of a nucleic acid encoded antigen and an immune response activator, such as a cytokine, is advantageous because it can induce activation and/or maturation of dendritic cells. Administration of such a composition can facilitate activation of dendritic cells and is essential for dendritic cells to cross-prime antigen to T-cells and generate effective immunity.

In some embodiments, a p-GlcNAc nanoparticle/nucleic acid composition is used for repeated administration. In some embodiments, the p-GlcNAc nanoparticle/nucleic acid composition is administered three times a day, two times a day, once a day, once in two days, once a week, once in two weeks or once a month for a period of one month, two months, three months, six months, one year, or more than one year. In other embodiments, a p-GlcNAc nanoparticle/nucleic acid composition is for one-time, non-recurring administration.

In some embodiments, a p-GlcNAc nanoparticle/nucleic acid composition comprising 0.1 μg, 0.5 μg, 1 μg, 1.5 μg, 2 μg, 3 μg, 4 μg, 5 μg, 6 μg, 7 μg, 8 μg, 9 μg, 10 μg, 15 μg, 20 μg, 25 μg, 30 μg, 35 μg, 40 μg, 45 μg, 50 μg, 60 μg, 75 μg, 80 μg, 90 μg, 100μg, 125 μg, 150 μg, 200 μg, 250 μg, 300 μg, 350 μg, 400 μg or 500 μg of nucleic acid is administered to a subject. In certain embodiments, a p-GlcNAc nanoparticle/nucleic acid composition comprising 0.1 μg to 2 mg, 0.2 μg to 1 mg, 0.5 μg to 500 μg, 1 μg to 200 μg, 1 μg to 100 μg, 1 μg to 50 μg, 5 μg to 25 μg, 5 μg to 15 μg, 50 μg to 150 μg, 1 μg to 5 μg, 2 μg to 5 μg, 1 μg to 10 μg, 5 μg to 10 μg, 5 μg to 15 μg, 10 μg to 15 μg, 10 μg to 20 μg, or 15 μg to 25 μg of nucleic acid is administered to a subject.

In some embodiments, a p-GlcNAc nanoparticle/nucleic acid composition is advantageous because it reduces the frequency of administration of its components, by allowing sustained release and/or expression of such components, while maintaining the therapeutic concentration of such components at a desired level.

The terms "subject" and "patient" are used interchangeably to refer to an animal, including a non-human animal and a human animal. In certain embodiments, a p-GlcNAc nanoparticle/nucleic acid composition is administered to a mammal which is 0 to 6 months old, 6 to 12 months old, 1 to 5 years old, 5 to 10 years old, 10 to 15 years old, 15 to 20 years old, 20 to 25 years old, 25 to 30 years old, 30 to 35 years old, 35 to 40 years old, 40 to 45 years old, 45 to 50 years old, 50 to 55 years old, 55 to 60 years old, 60 to 65 years old, 65 to 70 years old, 70 to 75 years old, 75 to 80 years old, 80 to 85 years old, 85 to 90 years old, 90 to 95 years old or 95 to 100 years old. In certain embodiments, the mammal is a non-human mammal. In some embodiments, the mammal is an animal model for a particular disorder. In certain embodiments, the mammal is at risk or prone to a particular disorder. In other embodiments, the mammal has been diagnosed as having a particular disorder. In some embodiments, the mammal manifests symptoms of a particular disorder.

In specific embodiments, a p-GlcNAc nanoparticle/nucleic acid composition is administered to a human. In certain embodiments, a p-GlcNAc nanoparticle/nucleic acid composition is administered to a human 0 to 6 months old, 6 to 12 months old, 1 to 5 years old, 5 to 10 years old, 5 to 12 years old, 10 to 15 years old, 15 to 20 years old, 13 to 19 years old, 20 to 25 years old, 25 to 30 years old, 20 to 65 years old, 30 to 35 years old, 35 to 40 years old, 40 to 45 years old, 45 to 50 years old, 50 to 55 years old, 55 to 60 years old, 60 to 65 years old, 65 to 70 years old, 70 to 75 years old, 75 to 80 years old, 80 to 85 years old, 85 to 90 years old, 90 to 95 years old or 95 to 100 years old. In some embodiments, the human is at risk or prone to a particular disorder. In other embodiments, the human has been diagnosed as having a particular disorder. In some embodiments, the human manifests symptoms of a particular disorder.

In certain embodiments, a p-GlcNAc nanoparticle/nucleic acid composition is administered to a pet, *e.g.*, a dog or cat. In certain embodiments, a p-GlcNAc nanoparticle/nucleic acid composition is administered to a farm animal or livestock, *e.g.*, pig, cows, horses, chickens, etc. In some embodiments, the pet, farm animal or livestock is at risk or prone to a particular disorder. In other embodiments, the pet, farm animal or livestock has been diagnosed as having a particular disorder. In some embodiments, the pet, farm animal or livestock manifests symptoms of a particular disorder.

In some embodiments, a p-GlcNAc nanoparticle/nucleic acid composition is administered to a subject who is refractory to a standard therapy. In some embodiments, a p-GlcNAc nanoparticle/nucleic acid composition is administered to a subject who is susceptible to adverse reactions to a conventional therapy or therapies.

In addition, p-GlcNAc nanoparticle/nucleic acid compositions can be used to transfect (e.g., stably transfect) cells to produce large quantities of the nucleic acid gene product suitable for *in vitro* and/or *in vivo* uses. In one embodiment, the cells used for delivery of the nucleic acids are cell lines. In another embodiment, the cells used for delivery of the nucleic acids are primary cells from a subject (preferably, a human subject). In a specific embodiment, the cells used for delivery of the nucleic acids are cancer cells. Cells transfected with the nucleic acid delivery composition may also be administered to a subject (preferably, a human subject) as part of a gene therapy protocol.

### 5.6 Kits

Provided herein is a pharmaceutical pack or kit comprising one or more containers filled with one or more of the ingredients to produce p-GlcNAc nanoparticle/nucleic acid composition. In a specific embodiment, a pharmaceutical pack or kit comprises a deacetylated poly-N-acetylglucosamine ammonium salt derivative (e.g., a lactate derivative), in a container. In certain embodiments, the pharmaceutical pack or kit also comprises one or more of the following: (i) sodium acetate-acetic acid buffer pH 5.7 *(e.g.,* 25 mM sodium acetate-acetic acid buffer pH 5.7), in a container; (ii) sodium sulfate (e.g., 50 mM sodium sulfate), in a container; (iii) a nucleic acid in a container; and (iv) an adjuvant in a container.

In certain embodiments, a pharmaceutical pack or kit comprises a deacetylated poly-N-acetylglucosamine ammonium salt derivative (e.g., a lactate derivative), a nucleic acid, and optionally, an adjuvant. In some embodiments, a pharmaceutical pack or kit comprises a deacetylated poly-N-acetylglucosamine ammonium salt derivative (e.g., a lactate derivative), a nucleic acid, and optionally, an adjuvant, wherein the poly-N-acetylglucosamine ammonium salt derivative is in a separate container from the nucleic acid and, optionally, the adjuvant. In some embodiments, a pharmaceutical pack or kit comprises a deacetylated poly-N-acetylglucosamine ammonium salt derivative (e.g., a lactate derivative), a nucleic acid, and an adjuvant, wherein each of the poly-N-acetylglucosamine ammonium salt derivative, the nucleic acid and the adjuvant is placed in a separate container. In other embodiments, the nucleic acid and the adjuvant are in the same container of the pharmaceutical pack or kit. In certain embodiments, the pharmaceutical pack or kit further comprises one or more of the following: (i) an acetic acid buffer such as ammonium acetate-acetic acid buffer or sodium acetate-acetic acid buffer (e.g., pH 5.7 such as 25 mM sodium acetate-acetic acid buffer pH 5.7), in a container; and/or (ii) sodium sulfate, potassium sulfate, calcium sulfate or magnesium sulfate (e.g., 50 mM sodium sulfate), in a container. In some embodiments, the poly-N-acetylglucosamine ammonium salt derivative is in the same container as an acetic acid buffer such as sodium acetate-acetic acid buffer or ammonium acetate-acetic acid buffer. In other embodiments, the poly-N-acetylglucosamine ammonium salt derivative is in a different container from an acetic acid buffer such as sodium acetate-acetic acid buffer or ammonium acetate-acetic acid buffer. In some embodiments, the nucleic acid is in the same container as sodium sulfate, potassium sulfate, calcium sulfate or magnesium sulfate. Yet in other embodiments, the nucleic acid is in a different container from sodium sulfate, potassium sulfate, calcium sulfate or magnesium sulfate.

Optionally associated with such container(s) can be a notice in the form prescribed by a governmental agency regulating the manufacture, use or sale of pharmaceuticals or biological products, which notice reflects approval by the agency of manufacture, use or sale for human administration.

The kits encompassed herein can be used in the above methods.

### 6. EXAMPLES

### 6.1 Example 1: Preparation of p-GlcNAc Nanoparticle/Nucleic Acid Composition

Step One: Determination of p-GlcNAc slurry concentration

     1.1 Dilute p-GlcNAc slurry stock to 20 liters with deionized (DI) water and mix overnight or 24 hours on shaker.
     1.2 Filter 10mL of the diluted slurry using Supro 800 filter membrane three times (30mL total volume). Incubate the three membranes in an 85°C oven until they are dried.
     1.3 Weigh the three membranes and take the average weight.
     1.4 Calculate the concentration by dividing the average weight by 10.

For example, the resulting p-GlcNAc slurry can have an average weight = 6.3mg, and concentration = 6.3mg/10mL= 0.63mg/mL

Step Two: Calculate volume needed to make mats
The dimension of the mat box is 22cm x 22cm, thus the area of the box is 484cm$^2$.

2.1 The amount of polymer that may be used or is required is 0.5mg/cm$^2$; therefore, the amount of polymer needed for one mat is 0.5mg/cm$^2$ x by 484 cm$^2$, which is 242mg.

2.2 The volume that may be used or is required for one mat is 242mg/0.63mg/mL, which is 384mL.

2.3 Pour 384mL of diluted slurry into the metal box with the metal screen, filter and remove the mat. Incubate the mat in a 50°C oven until dry or let it dries at room temperature on paper towels.

Step Three: Membrane (Mat) Deacetylation

3.1 Make 40% Sigma Sodium Hydroxide (NaOH flakes) solution one day prior to deacetylation reaction because the solution takes 24 hours to cool down. This is a weight to volume formulation; therefore, 40grams of NaOH flakes per 60mL of DI water (weight to volume). Once the solution is cool, pour into a 1 liter bottle.

3.2 Turn the water bath ON and set it to 80°C. Soak the membrane in 40% NaOH solution to loosen it from the screen and transfer it into a 1-liter glass bottle. Place the metal screen into a 4-liters beaker. Once all the membranes are transferred into the glass bottle, fill the bottle with the remaining 40% NaOH solution above the 1,000mL mark and place the bottle in the water bath.

3.3 Incubate the bottle with membranes for 3 hours. Remove and shake the bottle every 30 minutes in order to mix it. Three hours of incubation will give you approximately 75% deacetylation measurement.

3.4 Remove the bottle and turn OFF the water bath. Let the membranes cool down, pour the 40% NaOH solution into a 4-liters flask and wash the membranes with DI water until the pH is neutral (7). Soak the membranes in DI water overnight and dispose of the 40% NaOH solution properly.

3.5 Place the deacetylated membranes on the metal screen and dry them in the 50°C oven and measure deacetylation.

Step Four: Measure Deacetylation Percentage

4.1 Make acetic acid standard (0.01, 0.02 and 0.03M) and glucosamine standard (0.005, 0.015 and 0.035mg/mL) and run them on the programmed spectrophotometer to obtain a standard curve.

4.2 Weigh two weights per sample between 0.5mg and 1.0mg. Dissolve the sample with 100μL of acetic acid for 20 minutes, bring the volume up to 1mL w/ 900μL of DI water. Aliquot 50μL, 100μL, 150μL of the sample into three eppendorf tubes containing 950μL, 900μL, 850μL of 0.01M acetic acid, mix well and read the sample in the spectrophotometer.

4.3 Calculate the deacetylation percentage using Excel spreadsheet once the standard and sample reading are obtained.

Step Five: After Deacetylation Measurement calculate the amount of lactic acid needed to make gel.
Example for 69% Deacetylated Membrane:

Acetyl Glucosamine     221.2 X .31 = 6857.2
Glucosamine            215.6 X .69 = 14876.4

$$\text{Sum} = \frac{21733.6}{100} = 217 - \text{average MW}$$

$$\text{Weight of polymer} \frac{10g}{217} = 0.046 \text{ M}$$

$$30\% \text{ Lactic Acid} = 3.33 \text{ M}$$

To achieve 1:1 molar ratio p-GlcNAc:LA

$$\frac{46}{3.33} = 13.81\text{mL}$$ of Lactic Acid needed for this sample.

Step Six: Pour 13.81mL of 30% Lactic Acid into beaker with 986 mL of DI water with DEAC membranes. Leave membranes stir overnight to obtain uniform solution. Filter gel material through glass filter. Freeze in -20°C freezer in plastic covered trays and lyophilize.

Step Seven: Dissolve 2g of lyophilized material in 100 ml of DI water to obtain 100 ml of 2% p-GlcNAc gel. Sterilize gel with autoclaving 120°C 20 min.

Step Eight: This protocol is scaled for 1 animal injection: (1) Dilute p-GlcNAc gel 100 times in 25 mM sodium acetate-acetic acid buffer pH 5.7 and place in a water bath 55°C for 15 min (final p-GlcNAc concentration after dilution is 0.02% gel). (2) Add 10 microgram of DNA plasmid to 100 micro liters of 50 mM sodium sulfate and place in a water bath at 55°C for 10 min. (3) Add 100 micro liters of diluted p-GlcNAc gel to the DNA-sodium sulfate solution, while sample is being vortexed at a high speed. (4) Continue vortex the mixture for 20 seconds. (5) Keep the mixture at room temperature before injection into a subject for injection into a subject for under 2 hours. The resulting p-GlcNAc nanoparticle/DNA composition is used for injection into a subject.

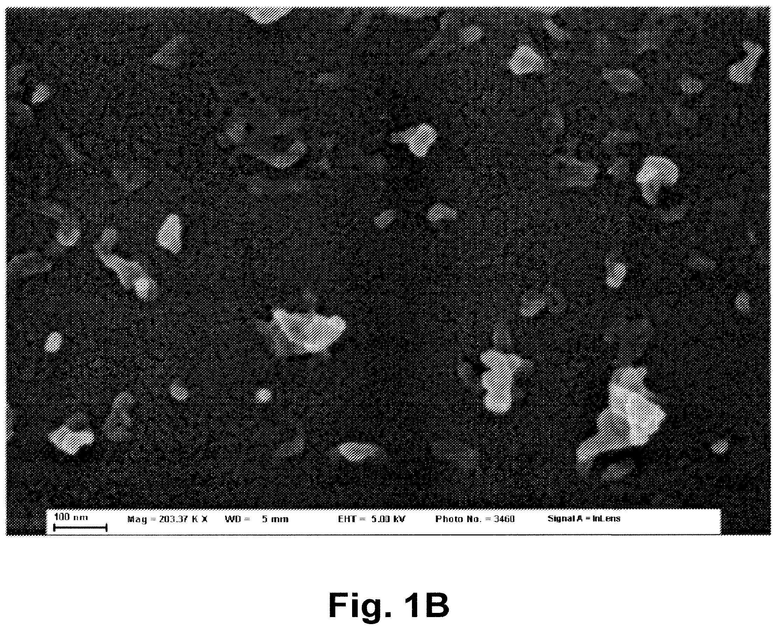

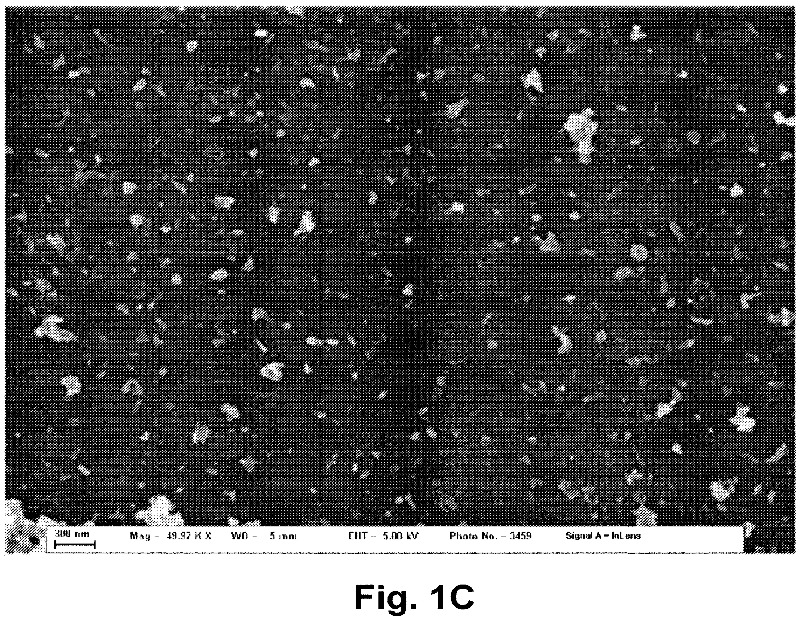

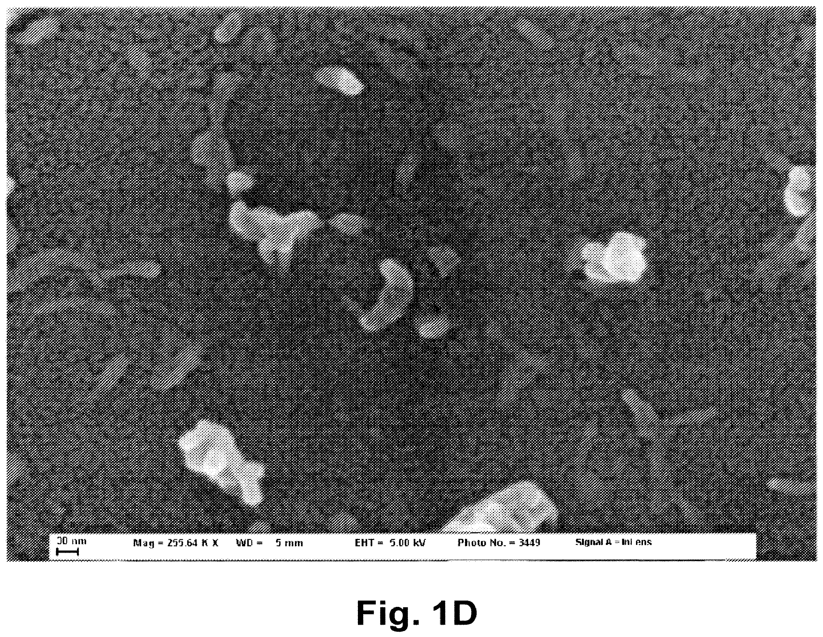

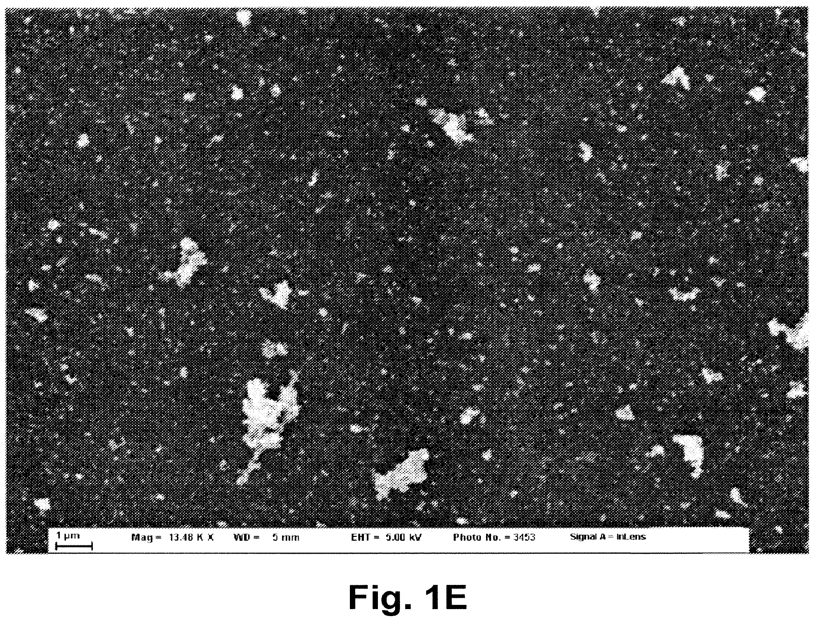

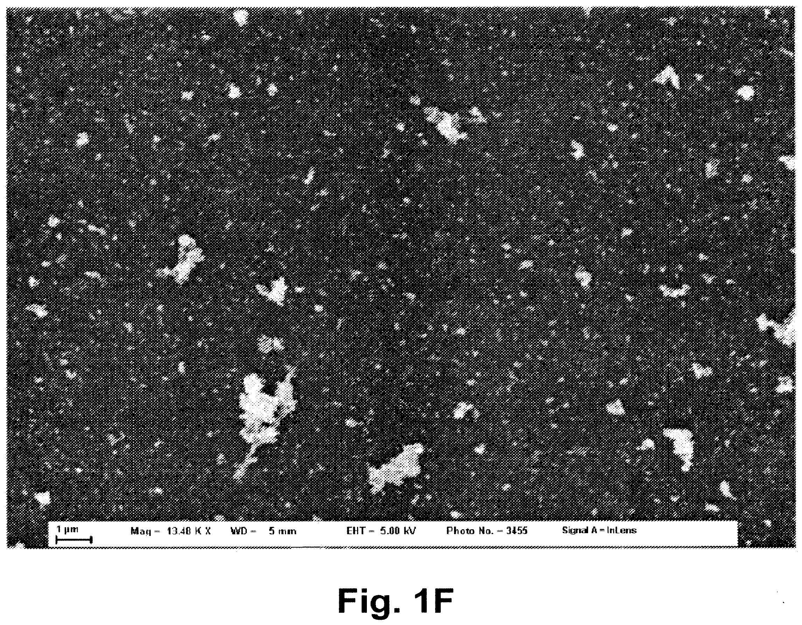

Figure 1 shows scanning electron micrographs of p-GlcNAc nanoparticles.

### 6.2 Example 2: *In vivo* DNA Vaccination Using Luciferase Gene With or Without p-GlcNAc Nanoparticle Composition.

The protocol referenced in section 6.1 was used to produce p-GlcNAc nanoparticle/DNA composition comprising plasmid DNA encoding luciferase. Plasmid preparations comprising DNA encoding luciferase (pcDNA*Luc*) were injected (100 μg/mouse) intramuscularly ("i.m") as naked DNA preparations or subcutaneously ("s.c") as either naked DNA or p-GlcNAc nanoparticle/DNA compositions. Luciferase activity was detected by bioluminescence imaging using the IVS system after intraperitoneal injection of luciferin substrate at days 1, 7 and 14 after administration of the DNA composition. Figure 2 shows the luciferase activity in all mice injected with pcDNA*Luc* compositions. The highest overall luciferase activity was detected in mice injected subcutaneously with p-GlcNAc nanoparticle/DNA compositions. Remarkably, DNA expression was detected in the same animals that received a single subcutaneous injection of the p-GlcNAc nanoparticle/DNA composition at comparable levels to mice that received an intramuscular injection up to four days after injection. Furthermore, Fig. 2 shows that transgene expression was detectable up to 14 days after vaccination with p-GlcNAc nanoparticle/DNA composition, which suggests a sustained availability of antigen locally at the site of administration. This data show that p-GlcNAc polymer nanoparticles are capable of releasing plasmid DNA in a way that results in sustained expression of the encoded antigen.

### 6.3 Example 3: Effective Uptake and Transport of DNA Encoded Antigen to Draining Lymph Node by Professional Antigen Presenting Cells Using p-GlcNAc Nanoparticle/DNA Composition

To determine whether DNA was effectively taken up by professional antigen presenting cells and transported to the draining lymph node, six mice were injected in the footpad with p-GlcNAc nanoparticle alone or an p-GlcNAc nanoparticle/DNA composition comprising 100 μg of plasmid DNA encoding GFP. The protocol in section 6.1 was used to generate the p-GlcNAc nanoparticle/DNA composition. Draining lymph nodes were excised one day after injection and cell suspensions were stained with monoclonal antibody against MHC ClassII conjugated with PE. The cell suspensions were analyzed by flow cytometry for dual expression of MHC Class II and green fluorescent protein (GFP). Figure 3 shows flow cytometry analysis of cell suspensions from draining lymph nodes of mice immunized with p-GlcNAc nanoparticle/pGFP compositions and of mice immunized with p-GlcNAc nanoparticle alone, wherein mice vaccinated with p-GlcNAc nanoparticle/DNA compositions showed GFP signal in ≅30% of MCH Class II positive cells from excised lymph nodes. This indicates that the p-GlcNAc nanoparticle is capable of delivering DNA to the local injection site resulting in the successful expression of the coded product which was then taken up and transported to draining lymph nodes by professional antigen presenting cells (APCs).

### 6.4 Example 4: Proliferation of Donor Pmel Cells in Response to hgp100 DNA Vaccination

The protocol in section 6.1 was used to produce p-GlcNAc nanoparticles comprising hgp100 DNA. Mice were vaccinated with naked hgp100 DNA (intramuscularly and subcutaneously), p-GlcNAc nanogparticle/hgp100 (subcutaneously) or left unvaccinated 24 hours after adoptive transfer of $10^6$ Pmel splenocytes (naive Pmel cells: CD8$^+$ T cells TCR transgenic for an epitope within human gp100 (i.e., hgp100)). Levels of circulating Pmel cells were determined by flow cytometry of blood samples. Figure 4 shows the proliferation of Pmel cells in response to vaccination with either naked hgp100DNA or p-GlcNAc nanoparticle/hgp100DNA in spleen, peripheral blood ("blood") and lymph nodes ("LN"). Higher frequencies of proliferating donor Pmel cells were found in the lymph nodes. p-GlcNAc nanoparticle/DNA compositions effectively activated antigen-specific CD8$^+$ T cell responses as evidenced by proliferation of naive Pmel cells in response to immunization with p-GlcNAc nanoparticle/phgp100 compositions.

### 6.5 Example 5: Co-Delivery of Poly I:C Enhances the Therapeutic Efficacy of DNA Vaccines Encoding Self Tumor Antigens

A previously established vaccination model that employs DNA encoding TRP2, a melanocyte differentiation antigen highly expressed in mouse and human melanomas was used. Previous studies have shown that the therapeutic efficacy of vaccination with naked DNA encoding TRP2 is minimal. Two experimental approaches, i.e., subcutaneous therapeutic model and metastasis therapeutic model, were utilized to test the efficacy of p-GlcNAc nanoparticle/DNA and p-GlcNAc nanoparticle/DNA/adjuvant compositions.

For the metastasis therapeutic model, five mice were injected intravenously with $3 \times 10^4$ B16 melanoma cells for each of the PBS saline control, p-GlcNAc nanoparticle/pDNA and p-GlcNAc nanoparticle/pDNA/Poly I:C. Mice were vaccinated subcutaneously three days apart (three vaccinations) starting at day 3 of the tumor injection with PBS saline, p-GlcNAc nanoparticle/pDNA or p-GlcNAc nanoparticle/pDNA/Poly I:C. All mice were sacrificed after tumor injection, and their lungs were excised and weighed. Figure 5A shows that the average lung weight of mice injected with p-GlcNAc nanoparticle/pDNA is lower than lung weight of mice injected with PBS saline. Remarkably, Figure 5A shows that the average lung weight of mice injected with p-GlcNAc nanoparticle/pDNA/adjuvant is significantly lower than lung weight of mice injected with either p-GlcNAc nanoparticle/DNA or PBS saline.

For the subcutaneous therapeutic model, five mice were injected subcutaneously with $10^5$ B16 melanoma cells for each of the PBS saline control, p-GlcNAc nanoparticle/pDNA and p-GlcNAc nanoparticle/pDNA/Poly I:C. Three subcutaneous vaccinations were given three days apart starting at day five after injection of tumor cells, with either saline, p-GlcNAc nanoparticle/pTRP2, or p-GlcNAc nanoparticle/pTRP2/ adjuvant (where adjuvant is Poly I:C). Tumor progression was monitored three times a week following treatment. Figure 5B shows the effect of the p-GlcNAc nanoparticle compositions on tumor size. Figure 5B demonstrates that p-GlcNAc nanoparticle/DNA composition inhibits tumor growth relative to saline control; it also demonstrates that p-GlcNAc nanoparticle/DNA/adjuvant composition shows greater inhibition of tumor growth than p-GlcNAc nanoparticle/DNA composition without an adjuvant.

Taken together, Figure 5 suggests that addition of adjuvant to the p-GlcNAc nanoparticle/DNA composition in the context of a therapeutic vaccination enhances antitumor immunity and delays tumor progression in both metastasis and subcutaneous models.

## Claims

1. A poly-N-acetylglucosamine nanoparticle/nucleic acid composition for use in a method of treating or preventing cancer, an infectious disease, or a genetic deficiency of a necessary protein,

   wherein the poly-N-acetylglucosamine nanoparticle/nucleic acid composition comprises poly-N-acetylglucosamine and the nucleic acid, wherein the nanoparticles are between 5 nm and 500 nm in size, and wherein 40% to 80% of the poly-N-acetylglucosamine is deacetylated; and wherein the method comprises administering the poly-N-acetylglucosamine nanoparticle/nucleic acid composition subcutaneously to the subject.

2. The poly-N-acetylglucosamine nanoparticle/nucleic acid composition for use of claim 1, wherein the subject is human.

3. The poly-N-acetylglucosamine nanoparticle/nucleic acid composition for use of claim 1, wherein the subject is a non-human animal.

4. The poly-N-acetylglucosamine nanoparticle/nucleic acid composition for use of any one of claims 1 to 3, wherein the administering of the poly-N-acetylglucosamine nanoparticle/nucleic acid composition results in a sustained expression of the nucleic acid for at least 1 week, 2 weeks, or 4 weeks at the site of administration to the subject.

5. The poly-N-acetylglucosamine nanoparticle/nucleic acid composition for use of any one of claims 1 to 4, wherein the method comprises repeated administration.

6. The poly-N-acetylglucosamine nanoparticle/nucleic acid composition for use of any one of claims 1 to 5, which further comprises an adjuvant, preferably the adjuvant is a cytokine or a polyinosinic:polycytidylic acid ("poly I:C").

7. The poly-N-acetylglucosamine nanoparticle/nucleic acid composition for use of claim 6, which provides sustained concurrent release of both the nucleic acid and the adjuvant.

8. The poly-N-acetylglucosamine nanoparticle/nucleic acid composition for use of any one of claims 1 to 7, wherein:

   (a) the deacetylated poly-N-acetylglucosamine comprises a deacetylated poly-N-acetylglucosamine ammonium salt derivative, preferably a deacetylated poly-N-acetylglucosamine lactate derivative; or
   (b) the deacetylated poly-N-acetylglucosamine has been solubilized with an organic or mineral acid, preferably with a lactic acid.

9. The poly-N-acetylglucosamine nanoparticle/nucleic acid composition for use of any one of claims 1 to 8, wherein at least 50% of the nanoparticles are between 20 nm and 200 nm, or between 25 nm and 150 nm in size.

10. The poly-N-acetylglucosamine nanoparticle/nucleic acid composition for use of any one of claims 1 to 9, wherein the size is determined by transmission electron microscopy or scanning electron microscopy.

11. The poly-N-acetylglucosamine nanoparticle/nucleic acid composition for use of any one of claims 1 to 10, wherein the method is part of a gene therapy protocol or vaccination protocol.

12. A method of making a poly-N-acetylglucosamine nanoparticle/nucleic acid composition comprising:

   (a) adding a base to poly-N-acetylglucosamine to deacetylate at least 40% to 80% of the poly-N-acetylglucosamine;
(b) adding a lactic acid to a form a deacetylated poly-N-acetylglucosamine lactate derivative;
(c) adding a buffer to facilitate dilution; and
(d) adding a nucleic acid, thereby making a poly-N-acetylglucosamine nanoparticle/nucleic acid composition.

**13.** The method of claim 12, wherein the buffer in step (c) is sodium acetate-acetic buffer.

**14.** The method of claim 12 or 13, wherein the nucleic acid has been combined with a salt, preferably sodium sulfate.

**15.** The method of any one of claims 12 to 14, which further comprises:

(i) adding an adjuvant in step (d); or
(ii) combining the poly-N-acetylglucosamine nanoparticle/nucleic acid composition with an adjuvant,

wherein the adjuvant is preferably poly I:C.

**16.** The poly-N-acetylglucosamine nanoparticle/nucleic acid composition for use of any one of claims 1 to 11, or the method of any one of claims 12 to 15, wherein the poly-N-acetylglucosamine is 60% to 80% deacetylated.

**17.** The poly-N-acetylglucosamine nanoparticle/nucleic acid composition for use of any one of claims 1 to 11, or the method of any one of claims 12 to 15, wherein the poly-N-acetylglucosamine is 65% to 75% deacetylated.

**18.** The poly-N-acetylglucosamine nanoparticle/nucleic acid composition for use of any one of claims 1 to 11, or 16 to 17, or the method of any one of claims 12 to 15 or 16 to 17, wherein the nucleic acid is DNA.

**Patentansprüche**

**1.** Poly-N-acetylglucosamin-Nanopartikel/Nukleinsäurezusammensetzung zur Verwendung in einem Verfahren zur Behandlung oder Prävention von Krebs, einer Infektionskrankheit oder einem genetischen Mangel an einem notwendigen Protein,

wobei die Poly-N-acetylglucosamin Nanopartikel/ Nukleinsäurezusammensetzung Poly-N-acetylglucosamin und die Nukleinsäure umfasst, wobei die Nanopartikel zwischen 5 nm und 500 nm groß sind und wobei 40% bis 80% des Poly-N-acetylglucosamins deacetyliert sind; und

wobei das Verfahren das Verabreichen der Poly-N-acetylglucosamin-Nanopartikel/Nukleinsäurezusammensetzung subkutan an den Patienten umfasst.

**2.** Poly-N-acetylglucosamin-Nanopartikel/Nukleinsäure-Zusammensetzung zur Verwendung nach Anspruch 1, wobei der Patient ein Mensch ist.

**3.** Poly-N-acetylglucosamin-Nanopartikel/Nukleinsäurezusammensetzung zur Verwendung nach Anspruch 1, wobei der Patient ein nichtmenschliches Lebewesen ist.

**4.** Poly-N-acetylglucosamin-Nanopartikel/Nukleinsäurezusammensetzung zur Verwendung nach einem der Ansprüche 1 bis 3, wobei die Verabreichung der Poly-N-acetylglucosamin-Nanopartikel/Nukleinsäurezusammensetzung zu einer anhaltenden Expression der Nukleinsäure für mindestens 1 Woche, 2 Wochen oder 4 Wochen am Ort der Verabreichung an den Patienten führt.

**5.** Poly-N-acetylglucosamin-Nanopartikel/Nukleinsäurezusammensetzung zur Verwendung nach einem der Ansprüche 1 bis 4, wobei das Verfahren eine wiederholte Verabreichung umfasst.

**6.** Poly-N-acetylglucosamin-Nanopartikel/Nukleinsäure-Zusammensetzung zur Verwendung nach einem der Ansprüche 1 bis 5, die weiter ein Adjuvans umfasst, wobei das Adjuvans vorzugsweise ein Cytokin oder eine Polyinosin:Polycytidylsäure ("Poly I:C") ist.

**7.** Poly-N-acetylglucosamin-Nanopartikel/Nukleinsäurezusammensetzung zur Verwendung nach Anspruch 6, die eine anhaltende gleichzeitige Freisetzung sowohl der Nukleinsäure als auch des Adjuvans bereitstellt.

**8.** Poly-N-acetylglucosamin-Nanopartikel/Nukleinsäurezusammensetzung zur Verwendung nach einem der Ansprüche 1 bis 7, wobei:

(a) das deacetylierte Poly-N-acetylglucosamin ein deacetyliertes Poly-N-acetylglucosamin-Ammoniumsalzderivat, vorzugsweise ein deacetyliertes Poly-N-acetylglucosamin-Laktatderivat, umfasst; oder
(b) das deacetylierte Poly-N-acetylglucosamin mit einer organischen Säure oder Mineralsäure, vorzugsweise mit einer Milchsäure, solubilisiert wurde.

**9.** Poly-N-acetylglucosamin-Nanopartikel/Nukleinsäurezusammensetzung zur Verwendung nach einem der Ansprüche 1 bis 8, wobei mindestens 50% der Nanopartikel zwischen 20 nm und 200 nm oder zwischen 25 nm und 150 nm groß sind.

10. Poly-N-acetylglucosamin-Nanopartikel/Nukleinsäurezusammensetzung zur Verwendung nach einem der Ansprüche 1 bis 9, wobei die Größe durch Transmissionselektronenmikroskopie oder Rasterelektronenmikroskopie bestimmt wird.

11. Poly-N-acetylglucosamin-Nanopartikel/Nukleinsäurezusammensetzung zur Verwendung nach einem der Ansprüche 1 bis 10, wobei das Verfahren Teil eines Gentherapieprotokolls oder Impfprotokolls ist.

12. Verfahren zur Herstellung einer Poly-N-acetylglucosamin-Nanopartikel/Nukleinsäurezusammensetzung, umfassend:

(a) Hinzufügen einer Base zu Poly-N-acetylglucosamin zum Deacetylieren von mindestens 40% bis 80% des Poly-N-acetylglucosamins;
(b) Hinzufügen einer Milchsäure zu einer Bildung eines deacetylierten Poly-N-acetylglucosamin-Laktatderivats;
(c) Hinzufügen eines Puffers, um die Verdünnung zu erleichtern; und
(d) Hinzufügen einer Nukleinsäure, wodurch eine Poly-N-acetylglucosamin-Nanopartikel/Nukleinsäure-Zusammensetzung hergestellt wird.

13. Verfahren nach Anspruch 12, wobei der Puffer in Schritt (c) Natriumacetat-Essigsäurepuffer ist.

14. Verfahren nach Anspruch 12 oder 13, wobei die Nukleinsäure mit einem Salz, vorzugsweise Natriumsulfat, kombiniert wurde.

15. Verfahren nach einem der Ansprüche 12 bis 14, das weiter umfasst:

(i) Hinzufügen eines Adjuvans in Schritt (d); oder
(ii) Kombinieren der Poly-N-acetylglucosamin-Nanopartikel/Nukleinsäurezusammensetzung mit einem Adjuvans,

wobei das Adjuvans vorzugsweise Poly I:C ist.

16. Poly-N-acetylglucosamin-Nanopartikel/Nukleinsäurezusammensetzung zur Verwendung nach einem der Ansprüche 1 bis 11 oder das Verfahren nach einem der Ansprüche 12 bis 15, wobei das Poly-N-acetylglucosamin zu 60% bis 80% deacetyliert ist.

17. Poly-N-acetylglucosamin-Nanopartikel/Nukleinsäure-Zusammensetzung zur Verwendung nach einem der Ansprüche 1 bis 11 oder das Verfahren nach einem der Ansprüche 12 bis 15, wobei das Poly-N-acetylglucosamin zu 65% bis 75% deacetyliert ist.

18. Poly-N-acetylglucosamin-Nanopartikel/Nukleinsäurezusammensetzung zur Verwendung nach einem der Ansprüche 1 bis 11 oder 16 bis 17 oder das Verfahren nach einem der Ansprüche 12 bis 15 oder 16 bis 17, wobei die Nukleinsäure DNA ist.

**Revendications**

1. Composition de nanoparticules de poly-N-acétylglucosamine/acides nucléiques pour utilisation dans une méthode de traitement ou de prévention du cancer, d'une maladie infectieuse, ou d'une déficience génétique d'une protéine nécessaire,
dans laquelle la composition de nanoparticules de poly-N-acétylglucosamine/acides nucléiques comprend de la poly-N-acétylglucosamine et des acides nucléiques, dans laquelle les nanoparticules présentent une taille entre 5 nm et 500 nm, et dans laquelle de 40% à 80% de la poly-N-acétylglucosamine est désacétylée; et
dans laquelle la méthode comprend une administration sous-cutanée de la composition de nanoparticules de poly-N-acétylglucosamine/acides nucléiques au sujet.

2. Composition de nanoparticules de poly-N-acétylglucosamine/acides nucléiques pour utilisation selon la revendication 1, dans lequel le sujet est un humain.

3. Composition de nanoparticules de poly-N-acétylglucosamine/acides nucléiques pour utilisation selon la revendication 1, dans lequel le sujet est un animal non humain.

4. Composition de nanoparticules de poly-N-acétylglucosamine/acides nucléiques pour utilisation selon l'une quelconque des revendications 1 à 3, dans laquelle l'administration des nanoparticules de poly-N-acétylglucosamine/acides nucléiques résulte en une expression prolongée des acides nucléiques pendant au moins 1 semaine, 2 semaines, ou 4 semaines au site d'administration du sujet.

5. Composition de nanoparticules de poly-N-acétylglucosamine/acides nucléiques pour utilisation selon l'une quelconque des revendications 1 à 4, dans laquelle la méthode comprend une administration répétée.

6. Composition de nanoparticules de poly-N-acétylglucosamine/acides nucléiques pour utilisation selon l'une quelconque des revendications 1 à 5, qui comprend en outre un adjuvant, de préférence l'adjuvant est une cytokine ou un acide polyinosinique:acide polycytidylique ("poly I:C").

7. Composition de nanoparticules de poly-N-acétylglucosamine/acides nucléiques pour utilisation selon la revendication 6, qui fournit une libération simultanée prolongée de l'acide nucléique et de l'adjuvant.

8. Composition de nanoparticules de poly-N-acétylglucosamine/acides nucléiques pour utilisation selon l'une quelconque des revendications 1 à 7, dans laquelle:

   (a) la poly-N-acétylglucosamine désacétylée comprend un dérivé de sel de poly-N-acétylglucosamine ammonium désacétylée, de préférence un dérivé de lactate de poly-N-acétylglucosamine désacétylée; ou
   (b) la poly-N-acétylglucosamine désacétylée a été solubilisée avec un acide organique ou minéral, de préférence avec l'acide lactique.

9. Composition de nanoparticules de poly-N-acétylglucosamine/acides nucléiques pour utilisation selon l'une quelconque des revendications 1 à 8, dans laquelle au moins 50% des nanoparticules présentent une taille entre 20 nm et 200 nm, ou entre 25 nm et 150 nm.

10. Composition de nanoparticules de poly-N-acétylglucosamine/acides nucléiques pour utilisation selon l'une quelconque des revendications 1 à 9, dans laquelle la taille est déterminée par microscopie électronique à transmission ou par microscopie électronique à balayage.

11. Composition de nanoparticules de poly-N-acétylglucosamine/acides nucléiques pour utilisation selon l'une quelconque des revendications 1 à 10, dans laquelle la méthode fait partie d'un protocole de thérapie génique ou d'un protocole de vaccination.

12. Procédé de préparation d'une composition de nanoparticules de poly-N-acétylglucosamine/acides nucléiques comprenant:

   (a) ajouter une base à la poly-N-acétylglucosamine pour désacétyler au moins de 40% à 80% de la poly-N-acétylglucosamine;
   (b) ajouter de l'acide lactique pour former un dérivé de lactate de poly-N-acétylglucosamine désacétylée;
   (c) ajouter un tampon pour faciliter la dilution; et
   (d) ajouter un acide nucléique, produisant ainsi une composition de nanoparticules de poly-N-acétylglucosamine/acides nucléiques.

13. Procédé selon la revendication 12, dans lequel le tampon dans l'étape (c) est un tampon acétate de sodium-acétique.

14. Procédé selon la revendication 12 ou 13, dans lequel l'acide nucléique a été combiné avec un sel, de préférence du sulfate de sodium.

15. Procédé selon l'une quelconque des revendications 12 à 14, qui comprend en outre :

   (i) ajouter un adjuvant à l'étape (d) ; ou
   (ii) combiner la composition de nanoparticules de poly-N-acétylglucosamine/acides nucléiques avec un adjuvant,

   dans lequel l'adjuvant est de préférence du poly I:C.

16. Composition de nanoparticules de poly-N-acétylglucosamine/acides nucléiques pour utilisation selon l'une quelconque des revendications 1 à 11, ou le procédé selon l'une quelconque des revendications 12 à 15, dans laquelle la poly-N-acétylglucosamine est désacétylée de 60% à 80%.

17. Composition de nanoparticules de poly-N-acétylglucosamine/acides nucléiques pour utilisation selon l'une quelconque des revendications 1 à 11, ou le procédé selon l'une quelconque des revendications 12 à 15, dans laquelle la poly-N-acétylglucosamine est désacétylée de 65% à 75%.

18. Composition de nanoparticules de poly-N-acétylglucosamine/acides nucléiques pour utilisation selon l'une quelconque des revendications 1 à 11, ou 16 à 17, ou le procédé selon l'une quelconque des revendications 12 à 15 ou 16 à 17, dans laquelle l'acide nucléique est un ADN.

Fig. 1A

Fig. 1B

Fig. 1C

**Fig. 1D**

Fig. 1E

Fig. 1F

EP 2 635 261 B1

**Fig. 2**

Fig. 3

Fig. 4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20100086613 A
- US 5622834 A
- US 5623064 A
- US 5624679 A
- US 5686115 A
- US 5858350 A
- US 6599720 B
- US 6686342 B
- US 7115588 B
- GB 2220211 A
- US 2007064857 W
- WO 2007109812 A
- US 2007064858 W
- WO 2007109813 A
- US 5057540 A

### Non-patent literature cited in the description

- **HARRINGTON et al.** *Hum. Gene Ther.,* 2002, vol. 13 (11), 1263-1280
- **HARRINGTON et al.** *J. Virol.,* 2002, vol. 76 (7), 3329-3337
- **BAROUCH et al.** *J. Virol.,* 2003, vol. 77 (13), 7367-7375
- **PREMENKO-LANIER et al.** *Virology,* 2003, vol. 307 (1), 67-75
- **RAMIREZ et al.** *J. Virol.,* 2000, vol. 74 (16), 7651-7655
- **RAMIREZ et al.** *J. Virol.,* 2000, vol. 74 (2), 923-933
- **TEWARY et al.** *J. Infect. Dis.,* 2005, vol. 191 (12), 2130-2137
- **MUZYCZKA.** *Curr. Top. Microbiol. Immunol.,* 1992, vol. 158, 97-129
- **VARMUS.** *Science,* 1988, vol. 240 (4858), 1427-1435
- **WASUNGU et al.** *J. Control. Release,* 2006, vol. 116 (2), 255-264
- **WASUNGU.** *Biochim. Biophys. Acta,* 2006, vol. 1758 (10), 1677-1684
- **MANSOURI et al.** *Eur. J. Pharm. Biopharm.,* 2004, vol. 57 (1), 1-8
- **HAMA.** *Mol. Ther.,* 2006, vol. 13 (4), 786-794
- **FORREST ; PACK.** *Mol. Ther..,* 2002, vol. 6 (1), 57-66
- **VOURNAKIS et al.** *J. Trauma,* 2004, vol. 57 (1), S2-6
- **KENSIL et al.** Vaccine Design: The Subunit and Adjuvant Approach. Plenum Press, 1995
- **STOUTE et al.** *N. Engl. J. Med.,* 1997, vol. 336, 86-91